# NEUE EUROPÄISCHE PATENTSCHRIFT

(11) **EP 3 490 644 B2**
(45) Veröffentlichungstag und Bekanntmachung des Hinweises auf die Entscheidung über den Einspruch: **13.11.2024**
(45) Hinweis auf die Patenterteilung: 26.05.2021
(21) Anmeldenummer: 17734223.5
(22) Anmeldetag: 21.06.2017
(51) Int. Cl.: A61M 5/32, A61M 5/20

(54) **ÄUSSERE KAPPE MIT NADELSCHUTZKAPPENENTFERNERELEMENT UND VERFAHREN ZUM MONTIEREN EINER INJEKTIONSVORRICHTUNG**
OUTER CAP WITH NEEDLE CAP REMOVER ELEMENT AND METHOD TO ASSEMBLE AN INJECTION DEVICE
CAPUCHON EXTERNE AVEC UN ÉLÉMENT POUR DÉCAPOUCHONNER UN CAPUCHON D'UNE SYRINGE ET UNE MÉTHODE D'ASSEMBLAGE D'UNE DISPOSITIF D'INJECTION

(30) Priorität: 28.07.2016 CH 9892016
(43) Veröffentlichungstag der Anmeldung: 05.06.2019
(73) Patentinhaber: Ypsomed AG, 3401 Burgdorf (CH)
(72) Erfinder: SCHRUL, Christian, 3400 Burgdorf (CH); TSCHIRREN, Markus, 3400 Burgdorf (CH); HUNZIKER, Markus, 4460 Gelterkinden (CH)
(74) Vertreter: Kleiner, Stefan
(86) Internationale Anmeldenummer: PCT/CH2017/000062
(87) Internationale Veröffentlichungsnummer: WO 2018/018165

(56) Entgegenhaltungen:
- EP-A1- 2 878 321
- EP-A1- 2 878 322
- EP-A1- 2 923 716
- WO-A1-2010/136076
- WO-A1-2015/110532
- US-A1- 2008 275 429
- US-A1- 2015 351 767

## Beschreibung

Die Erfindung betrifft eine Injektionsvorrichtung zur Verabreichung eines flüssigen Produkts, insbesondere eines Medikaments. Insbesondere betrifft die Erfindung einen Mechanismus für die Injektionsvorrichtung, mit dem eine an einem Produktbehälter angebrachte Nadelschutzkappe von dem Produktbehälter lösbar oder entfernbar ist. Des Weiteren betrifft die Erfindung ein Verfahren zum Montieren einer Injektionsvorrichtung und/oder zum Vorbereiten einer Injektionsvorrichtung für die Verabreichung eines Produkts.

Der Begriff "Medikament" umfasst hier jede fließfähige medizinische Formulierung welche geeignet ist zur kontrollierten Verabreichung durch ein Mittel wie eine Kanüle oder Hohlnadel hindurch, beispielsweise umfassend eine Flüssigkeit, eine Lösung, ein Gel, oder eine feine Suspension welche einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form aber auch Polysaccharide, Vaccine, DNS oder RNS oder Oglionukleotide, Antikörper oder Teile von Antikörpern sowie geeignete Basis-, Hilfs- und Trägerstoffe.

Aus dem Stand der Technik sind Injektionsvorrichtungen bekannt, in denen eine Fertigspritze angeordnet ist. Die Fertigspritze weist eine Injektionsnadel auf, die unlösbar mit der Fertigspritze verbunden ist und über die ein in der Fertigspritze enthaltenes Medikament ausgegeben werden kann. Um die Injektionsnadel der Fertigspritze steril zu halten, wird sie von einer an der Fertigspritze befestigten Nadelschutzkappe umschlossen und in Bezug auf die Umgebung steril abgedichtet. Solche Nadelschutzkappen können z. B. als sogenannte soft needle shield oder als rigid needle shield ausgestaltet sein. Ein soft needle shield besteht aus einem elastomeren Teil, welches die Nadel umgibt. Ein rigid needle shield weist mehrere Teile auf, insbesondere ein elastomeres kappenförmiges Teil und ein aus einem festen, d. h. nicht-elastomeren Kunststoff hergestelltes hülsenförmiges Teil, welches das elastomere Teil aufnimmt und damit im Wesentlichen unlösbar verbunden ist.

Bei der Handhabung der Fertigspritze besteht die Gefahr, dass durch eine Krafteinwirkung auf die Nadelschutzkappe die Sterilität der Nadel gefährdet wird. Dies kann insbesondere während des Montageprozesses der Injektionsvorrichtung, insbesondere wenn die Fertigspritze in ihren dafür vorgesehenen Spritzenhalter der Injektionsvorrichtung eingesetzt wird, auftreten. Das Einfügen der Fertigspritze in die Injektionsvorrichtung ist daher aus Sicht der Sterilität der Nadel ein Schritt, dem besondere Aufmerksamkeit gilt. Aus der WO 2010/136076 A1 ist es bekannt, dass beim Abziehen eines kappenförmigen Abziehelements, die auch als Kappe bezeichnet wird, am distalen Ende der Injektionsvorrichtung angebracht ist und das distale Ende der Injektionsvorrichtung verschließt, die an der Spritze angebrachte Nadelschutzkappe mitabgezogen, d. h. beim Entfernen der Kappe von der Spritze entfernt wird. Die Nadelschutzkappe verbleibt dabei in der Kappe. Hierzu weist die Kappe Eingriffsglieder auf, die beim Abziehen der Kappe in einen Eingriff mit der Nadelschutzkappe gebracht werden. Beim Fortsetzen der Abziehbewegung des Abziehelements nehmen die Eingriffsglieder die Nadelschutzkappe mit, wodurch diese vom Produktbehälter abgezogen wird. Um ein sicheres Abziehen der Nadelschutzkappe durch das Entfernen der Kappe zu gewährleisten, ist es aus dem Stand der Technik bekannt, dass sich mit der Kappe verbundene krallenförmige Elemente aus Metall in den Umfang der Nadelschutzkappe einkrallen. Dieses Einkrallen geschieht schon während der Montage, da die Nadelschutzkappe während der Montage der Injektionsvorrichtung in den Bereich der krallenförmigen Elemente gesteckt wird. Die krallenförmige Elemente üben während der Montage jedoch eine gewisse Kraft auf die Nadelschutzkappe aus, wodurch nicht ausgeschlossen werde kann, dass die Nadelschutzkappe in Bezug auf den Produktbehälter bewegt wird, wodurch die Sterilität der Injektionsnadel gefährdet sein kann.

Des Weiteren offenbaren EP 2878321 A1, EP 2878322 A1 und EP 2923716 A1 Abziehelemente, wobei beim Abziehen des Abziehelements von einer Injektionsvorrichtung eine Nadelschutzkappe von einer in der Injektionsvorrichtung aufgenommenen Spritze abgezogen wird.

Es ist somit eine Aufgabe der vorliegenden Erfindung, eine alternative Injektionsvorrichtung mit einer Kappe und einem Produktbehälter, wobei an dem Produktbehälter eine Nadelschutzkappe vorgesehen ist, bereitzustellen, wobei beim Entfernen der Kappe von der Injektionsvorrichtung das Entfernen einer Nadelschutzkappe von einem Produktbehälter bewirkt wird.

Die Aufgabe wird mit der Injektionsvorrichtung nach Anspruch 1 gelöst. Vorteilhafte Weiterbildungen ergeben sich aus den abhängigen Ansprüchen, der Beschreibung und den Figuren.

Die Erfindung geht von einer Vorrichtung zur Verabreichung eines Produkts, nämlich von einer Injektionsvorrichtung aus. Die Injektionsvorrichtung kann als so genannter Autoinjektor ausgestaltet sein, der einen Mechanismus aufweist, der ein automatisches Ausschütten des Produkts, wie z. B. durch einen Energiespeicher, insbesondere eine Feder, und optional ein automatisches Einstechen und/oder Zurückziehen der Nadel bewirkt. Bei einem Autoinjektor wird die Kraft zum Ausschütten des Produkts durch den Energiespeicher, wie z.B. die Feder bereitgestellt. Die Injektionsvorrichtung kann alternativ als manuelle Injektionsvorrichtung ausgestaltet sein, d. h., dass die Kraft für die Ausschüttung des Produkts durch Muskelkraft, wie z. B. durch den Benutzer selbst erfolgt. Die Injektionsvorrichtung - egal ob es sich um einen Autoinjektor oder eine manuelle Injektionsvorrichtung handelt - kann eine Nadelschutzhülse aufweisen, die nach erfolgter Injektion distal über das distale Ende der Injektionsnadel steht oder relativ zu dem Gehäuse in diese Position verschoben wird, um den versehentlichen Zugriff auf die Injektionsnadel zu verhindern und dadurch ein Verletzungsrisiko zu verringern. Bei einem Autoinjektor kann die Nadelschutzhülse beispielsweise auch als Auslöseelement zum Auslösen der Produktausschüttung dienen, wobei die Nadelschutzhülse hierzu relativ zu dem Gehäuse in die proximale Richtung verschoben wird.

Die Injektionsvorrichtung weist einen Produktbehälter mit einer Injektionsnadel auf, wie z. B. eine aus dem Stand der Technik bekannte Fertigspritze oder allgemein Spritze. Der Produktbehälter kann einen z. B. hohlzylindrischen Produktbehälterabschnitt aufweisen, der einen Kolben verschiebbar lagert. Der Kolben kann mit dem Innenumfang des Produktbehälterabschnitts einen Dichtspalt bilden. Der Kolben kann z. B. mittels einer Kolbenstange der Injektionsvorrichtung in die distale Richtung verschoben werden, um über die Injektionsnadel Produkt aus dem Produktbehälter auszugeben. Die Injektionsnadel kann z. B. unlösbar an dem Produktbehälter gebildet sein. Zum Beispiel kann der Produktbehälter einen Halteabschnitt, insbesondere einen Nadelhalteabschnitt, aufweisen, der distal des Produktbehälterabschnitts angeordnet ist und mit der Injektionsnadel unlösbar verbunden ist, wie zum Beispiel einen proximalen Teil der Nadel umgibt. Die Injektionsnadel kann somit von dem Halteabschnitt in die distale Richtung abragen. Der Halteabschnitt kann beispielsweise einen geringeren Außendurchmesser als der Produktbehälterabschnitt aufweisen. Der Produktbehälterabschnitt kann sich an seinem distalen Ende zu dem Halteabschnitt hin verjüngen.

Der hierin verwendete Begriff "distal" bezieht sich auf die Richtung, in die die Spitze der Injektionsnadel zeigt. Der hierin verwendete Begriff "proximal" bezieht sich auf die Richtung, die der distalen Richtung entgegengesetzt ist.

An dem Produktbehälter, beispielsweise an dem Halteabschnitt, ist eine Nadelschutzkappe, wie z. B. ein aus dem Stand der Technik bekanntes soft needle shield oder rigid needle shield, befestigt, insbesondere lösbar befestigt. Die Nadelschutzkappe kann z. B. reib- oder formschlüssig oder kombiniert reib- und formschlüssig auf dem Halteabschnitt befestigt sein. Die Nadelschutzkappe umschließt die Injektionsnadel und dichtet sie in Bezug auf die Umgebung steril ab. Ein soft needle shield umfasst oder besteht aus einem elastomeren, beispielsweise auf Gummi- oder Kautschukbasis gebildeten Teil, welches die Nadel umgibt. Das soft needle shield weist an seinem Außenumfang eine weiche, wie z. B. aus einem gummi- oder kautschukartigen Material gebildete Oberfläche auf. Ein rigid needle shield weist zumeist mehrere Teile auf, insbesondere ein elastomeres kappenförmiges inneres Teil und ein aus einem festen, d. h. nicht-elastomeren Kunststoff hergestelltes hülsenförmiges oder kappenförmiges äußeres Teil, welches das elastomere Teil aufnimmt und damit im Wesentlichen unlösbar verbunden ist. Das äußere hülsen- oder kappenförmige Teil umgibt das innere kappenförmige Teil und ist mit der inneren Kappe beispielsweise unlösbar verbunden, so dass die äußere und innere Kappe eine Einheit bilden. Das innere Teil kann aus einem härteren Kunststoff gebildet als das innere Teil. Das äußere Teil kann beispielsweise aus Polyethylen, Polystyrol, Polypropylen oder einem anderen geeigneten Kunststoff sein. Das innere Teil kann beispielsweise aus Gummi oder Kautschuk oder einem anderen geeigneten Material gebildet sein.

An dem distalen Ende der Injektionsvorrichtung oder eines Gehäuses, wie z. B. eines Aufnahmegehäuses der Injektionsvorrichtung kann eine Kappe, die auch als Verschlusskappe oder Abziehkappe bezeichnet werden oder ausgestaltet sein kann, befestigt sein und das distale Ende des Gehäuses oder des Aufnahmegehäuses verschließen. Die Kappe kann z. B. mit dem Gehäuse oder Aufnahmegehäuse reib- und/oder formschlüssig verbunden sein, wie z. B. verschnappt sein. Die Kappe kann z. B. während des Entfernens von der Injektionsvorrichtung oder dem Gehäuse mit einer Axialbewegung oder einer kombinierten Axial-DrehBewegung von der Injektionsvorrichtung, wie z. B. dem Gehäuse oder Aufnahmegehäuse, abnehmbar sein.

Die Kappe, welche mit einem oder mehreren Eingriffselementen gekoppelt ist, ist über das mindestens eine Eingriffselement so mit der Nadelschutzkappe verbunden, dass das Entfernen der Kappe von der Injektionsvorrichtung das Entfernen der Nadelschutzkappe von dem Produktbehälter bewirkt. Insbesondere kann zumindest ein Teil der Bewegung auf das Eingriffselement übertragen werden, d. h., dass das Eingriffselement von der Kappe mitgenommen wird, so dass das Eingriffselement die Nadelschutzkappe von dem Produktbehälter, insbesondere dem Halteabschnitt, abzieht.

Das mindestens eine Eingriffselement wird von einem beispielsweise hülsenförmigen Entfernerelement gebildet, welches an der Kappe befestigt ist. Die Kappe kann das beispielsweise hülsenförmige Entfernerelement umgeben, vorzugsweise über seinen Umfang einfassen. Das Entfernerelement ist vorzugsweise ein von der Kappe separates Element, welches jedoch mit der Kappe verbunden, wie z. B. entlang der Längsachse verschiebbar oder unverschiebbar verbunden sein kann.

Das Eingriffselement kann z. B. im Auslieferungszustand der Injektionsvorrichtung in Bezug auf die Nadelschutzkappe in einer Eingriffsposition sein. In der Eingriffsposition ist das Eingriffselement in Bezug auf die Nadelschutzkappe so angeordnet, dass eine Bewegung des Entfernerelements in die distale Richtung eine Mitnahme der Nadelschutzkappe bewirkt und somit die Nadelschutzkappe von dem Produktbehälter entfernt. In der Eingriffsposition ist ein Abschnitt der Nadelschutzkappe distal in einer Flucht mit dem Eingriffselement angeordnet. Das heißt, dass ein Teil der Nadelschutzkappe aus Sicht des Eingriffselements in distaler Richtung vor dem Eingriffselement angeordnet ist, wodurch die Bewegung des Eingriffselements in die distale Richtung die Mitnahme der Nadelschutzkappe bewirkt. Zum Beispiel kann das Eingriffselement an oder in die Nadelschutzkappe an- oder eingreifen, wenn es in der Eingriffsposition ist.

Die Kappe und das Eingriffselement sind so gekoppelt, dass die Kappe während des Entfernens von der Injektionsvorrichtung, insbesondere dem Gehäuse oder dem Aufnahmegehäuse, relativ zu dem Eingriffselement bewegbar ist oder bewegt wird. Der sich hierdurch ergebende Vorteil ist, dass durch diese Bewegung weitere Teile oder Abschnitte der Injektionsvorrichtung so positioniert werden können, dass die Verbindung des Entfernerelements mit der Nadelschutzkappe gesichert oder sogar verstärkt wird, wodurch sichergestellt wird, dass die Nadelschutzkappe beim Entfernen der Kappe sicher von dem Produktbehälter entfernt wird. Darüber hinaus besteht der Vorteil, dass die Teile oder Abschnitte des Entfernerelements, wie zum Beispiel das mindestens eine Eingriffselement, so ausgebildet sein können, dass die von ihnen beim Einfügen des Produktbehälters auf die Nadelschutzkappe ausgeübten Kräfte gering sind. Hierdurch wird verhindert, dass die Nadelschutzkappe bereits während des Einfügens des Produktbehälters relativ zu dem Produktbehälter bewegt wird. Dadurch wird die Gefahr verringert, dass die Sterilität der Nadel leidet.

Beispielsweise können die Kappe und das Eingriffselement so gekoppelt sein, dass die Kappe während des Entfernens von der Injektionsvorrichtung zunächst relativ zu dem Eingriffselement bewegbar ist oder bewegt wird und anschließend das Eingriffselement die Bewegung der Kappe mitmacht, wie z. B. von der Kappe oder der Bewegung der Kappe mitgenommen wird, wodurch die Nadelschutzkappe von dem Produktbehälter entfernt oder abgezogen wird. Mit anderen Worten umfasst der Hub, den die Kappe beim Entfernen von der Injektionsvorrichtung relativ dem Gehäuse entlang der Längsachse in die distale Richtung ausführt, einen ersten Teilhub, während dem die Kappe relativ zu dem Eingriffselement bewegbar ist oder bewegt wird, und einen zweiten Teilhub, während dem das Eingriffselement die Bewegung der Kappe mitmacht oder von der Kappe mitgenommen wird. Während des ersten Teilhubs kann das Eingriffselement derart gesichert werden, dass es nicht mehr aus seiner Eingriffsposition bewegbar ist, wobei die Nadelschutzkappe während des zweiten Teilhubs von dem Produktbehälter entfernt wird und z. B. die Injektionsnadel freigibt.

Die Injektionsvorrichtung weist einen Blockierabschnitt auf, der aus einer Freigabeposition in eine Blockierposition bewegbar ist oder bewegt wird, insbesondere während des ersten Teilhubs oder wenn die Kappe während des Entfernens von der Injektionsvorrichtung relativ zu dem Eingriffselement bewegt wird. Der Blockierabschnitt ist so ausgebildet, dass er in die Bewegbarkeit des Eingriffselements aus seiner Eingriffsposition oder/und eine Bewegung quer zur Längsachse nach außen, d.h. von der Längsachse weg, blockiert, wenn der Blockierabschnitt in einer Blockierposition ist. Der Blockierabschnitt befindet sich in der Freigabeposition, wenn die Kappe an dem Gehäuse, insbesondere dem Aufnahmegehäuse, angebracht, insbesondere vollständig angebracht oder befestigt ist. Der Blockierabschnitt ist so ausgestaltet, dass er die Bewegbarkeit des Eingriffselements quer nach außen nicht blockiert, wenn der Blockierabschnitt in seiner Freigabeposition ist. Der Blockierabschnitt ist mit der Kappe so gekoppelt, dass das Entfernen der Kappe von dem Gehäuse, insbesondere dem Aufnahmegehäuse, den Blockierabschnitt aus der Freigabeposition in die Blockierposition bewegt, insbesondere während des ersten Teilhubs oder wenn die Kappe relativ zu dem Eingriffselement bewegt wird, insbesondere während der Bewegung der Kappe entlang der Längsachse in die distale Richtung.

Beispielsweise kann der Blockierabschnitt eine nach innen oder zu der Längsachse hin weisende Fläche aufweisen, welche in der Blockierposition entlang der Längsachse auf Höhe oder in der Position des Eingriffselements angeordnet ist, wodurch das Eingriffselement gehindert wird, sich quer zur Längsachse nach außen zu bewegen. Insbesondere befindet sich das Eingriffselement zwischen dem Blockierabschnitt und der Längsachse der Injektionsvorrichtung, wenn der Blockierabschnitt in der Blockierposition ist.

Beispielsweise kann die Kappe eine Ausnehmung aufweisen, die in der Freigabeposition des Blockierabschnitts entlang der Längsachse in einer Position auf Höhe des oder in Position des Eingriffselements ist, wobei der Blockierabschnitt durch das Entfernen der Kappe in die Blockierposition bewegt wird, insbesondere während des ersten Teilhubs. Insbesondere kann das Eingriffselement zwischen der Ausnehmung und der Längsachse angeordnet sein, wenn der Blockierabschnitt in der Freigabeposition ist. Insbesondere kann die Ausnehmung so ausgestaltet sein, dass sie eine Bewegung des Eingriffselements quer zur Längsachse nach außen nicht verhindern oder blockieren kann.

Insbesondere kann die Kappe den Blockierabschnitt bilden oder aufweisen, wobei bevorzugt ist, dass der Blockierabschnitt proximal der Ausnehmung angeordnet ist.

Wenn der Blockierabschnitt beispielsweise von dem Entfernerelement gebildet ist, können der Blockierabschnitt und das Eingriffselement über einen Verformungsabschnitt verbunden sein, der so ausgestaltet ist, dass er sich während der Bewegung des Blockierabschnitts aus der Freigabeposition in die Blockierposition elastisch oder plastisch verformt. Insbesondere kann der Verformungsabschnitt flexibel sein. Der Verformungsabschnitt kann z. B. einen Koppelabschnitt aufweisen, der mit der Kappe axial fest oder begrenzt entlang der Längsachse axial verschiebbar gekoppelt oder in einem Eingriff ist. Der Verformungsabschnitt kann den Koppelabschnitt und das Eingriffselement verbinden. Der Verformungsabschnitt kann z. B. wendelförmig oder mäanderförmig ausgebildet sein, wodurch er entlang der Längsachse verformbar ist. Der Blockierabschnitt kann z. B. starr mit dem Koppelabschnitt verbunden sein, wodurch der Koppelabschnitt und der Blockierabschnitt nicht oder nicht nennenswert entlang der Längsachse relativ zueinander bewegt werden können.

Allgemein kann das z. B. hülsenförmige Entfernerelement einen Koppelabschnitt aufweisen, der in einem axial festen oder begrenzt axial verschiebbaren Eingriff mit der Kappe sein kann. Zum Beispiel kann der Koppelabschnitt eine Ausnehmung aufweisen, in welche eine Abragung der Kappe eingreift.

Der Blockierabschnitt ist in seiner Freigabeposition proximal des Eingriffsglieds und/oder des proximalen Endes der Nadelschutzkappe angeordnet.

In bevorzugten Ausführungsformen kann der Verformungsabschnitt oder das Eingriffselement einen Axialanschlag aufweisen, gegen den der Blockierabschnitt stößt, wenn er aus seiner Freigabeposition in die Blockierposition bewegt wird oder die Kappe von der Injektionsvorrichtung entfernt wird. Das Anschlagelement kann z. B. eine Nase sein, welche nach außen, d. h. von der Längsachse weg ragt und z. B. in einer axialen Flucht entlang der Längsachse mit dem Blockierabschnitt angeordnet ist.

In alternativen Ausführungen kann das Entfernerelement, insbesondere der Koppelabschnitt oder die Ausnehmung des Koppelabschnitts, in die die Kappe eingreift, einen Axialanschlag aufweisen, gegen den die Kappe, wie zum Beispiel die Abragung am Ende des ersten Teilhubs anschlägt, wodurch die Kappe das Entfernerelement nach Beendigung des ersten Teilhubs mitnehmen kann.

In Ausführungen, die nicht Teil der Erfindung sind, können die Kappe und das Entfernerelement in einem Eingriff sein, der so gestaltet ist, dass er bewirkt, dass ein Koppelabschnitt des Entfernerelements, der über einen Verformungsabschnitt, der die Nadelschutzkappe über ihre Umfangsfläche umgibt, mit dem Eingriffselement verbunden ist, beim oder während des Entfernens der Kappe von dem Aufnahmegehäuse von der Kappe mitgenommen wird. Dadurch kann sich der Koppelabschnitt relativ zu dem Eingriffselement bewegen und der Verformungsabschnitt derart verformt werden, dass sich der Verformungsabschnitt gegen die Umfangsfläche der Nadelschutzkappe einschnürt oder gegen die Umfangsfläche der Nadelschutzkappe drückt. Dadurch kann z. B. die Reibung zwischen der Nadelschutzkappe und dem Verformungsabschnitt erhöht und/oder ein Formschluss zwischen der Nadelschutzkappe und dem Verformungsabschnitt hergestellt werden. Hierdurch wird die für das Abziehen der Nadelschutzkappe von der Kappe auf die Nadelschutzkappe ausgeübte Kraft nicht oder nicht nur von dem Eingriffselement auf die Nadelschutzkappe übertragen sondern insbesondere zusätzlich über den Verformungsabschnitt.

Zum Beispiel kann der Verformungsabschnitt eine oder mehrere Eingriffsnocken aufweisen, die durch das Einschnüren des Verformungsabschnitts in die Umfangsfläche der Nadelschutzkappe eindringen. Alternativ oder zusätzlich kann der Verformungsabschnitt mindestens eine, wie z. B. eine einzige, zwei, drei, vier oder noch mehr Wendeln aufweisen, die sich jeweils mit einer oder mehreren Windungen um die Längsachse der Injektionsvorrichtung winden und den Koppelabschnitt mit dem Eingriffselement verbinden. Durch die wendelförmige Gestaltung des Verformungsabschnitts wird dessen Einschnürung, d. h. Innendurchmesserverringerung, bewirkt, wenn der Abstand zwischen dem Koppelabschnitt und dem Eingriffselement entlang der Längsachse vergrößert wird. Benachbarte Windungen der Wendeln können z. B. über einen oder mehrere Verbindungsstege verbunden sein. Die Stege haben den Effekt, dass die Grundstabilität des Entfernerteils erhöht wird - bspw. für Schüttguttauglichkeit und Montierbarkeit.

Ohne diese Verbindungsstege wäre der Verformungsabschnitt extrem flexibel (ähnlich einer Feder), was die Montage des Entfernerteils extrem erschweren würde
Ferner wird ein Verfahren zum Montieren einer Injektionsvorrichtung und/oder zum Vorbereiten einer Injektionsvorrichtung für die Verabreichung eines Produkts beschrieben, wobei besagtes Verfahren nicht Teil der Erfindung ist. Bei der Injektionsvorrichtung kann es sich z. B. um die hierin beschriebene Injektionsvorrichtung handeln.

Das Verfahren umfasst den Schritt des Bereitstellens eines Aufnahmegehäuses, welches z. B. Teil eines Gehäuses der Injektionsvorrichtung sein kann, zur Aufnahme eines Produktbehälters. Das Aufnahmegehäuse kann z. B. hülsenförmig und/oder länglich ausgestaltet sein. An einem distalen Ende des Aufnahmegehäuses kann die Kappe angebracht sein, wie z. B. mit dem Aufnahmegehäuse verschnappt sein, wobei in der Kappe das Entfernerelement angeordnet ist, das das quer zu der Längsachse des Aufnahmegehäuses bewegbare Eingriffselement aufweist.

Das Verfahren umfasst ferner den Schritt des Bereitstellens des Produktbehälters, der einen Produktbehälterabschnitt und eine mit dem Produktbehälterabschnitt verbundene Injektionsnadel aufweist, wobei der Produktbehälterabschnitt einen Kolben verschiebbar lagert, wobei zwischen der Injektionsnadel und dem Kolben das Produkt angeordnet ist. An dem Produktbehälter ist die Nadelschutzkappe angeordnet, welche die Injektionsnadel umschließt und gegenüber der Umgebung vorzugsweise steril abdichtet.

Das Verfahren umfasst ferner das Verschieben des Produktbehälters mitsamt der daran befestigten Nadelschutzkappe in dem und relativ zu dem Aufnahmegehäuse entlang der Längsachse in die distale Richtung. Optional kann der Produktbehälter mitsamt der daran befestigten Nadelschutzkappe über das proximale Ende, an dem das Aufnahmegehäuse eine Öffnung aufweist, in das Aufnahmegehäuse eingeführt werden, insbesondere mit der Nadelschutzkappe voran.

Während des Verschiebens des Produktbehälters mitsamt Nadelschutzkappe ist oder wird das Eingriffselement des Entfernerelements von einer Aussenfläche oder Aussenumfangsfläche der Nadelschutzkappe ausgelenkt. Da das insbesondere hülsenförmige Entfernerelement mehrere, wie z.B. zwei Eingriffselemente aufweist, die über den Umfang gegenüberliegen, können die Eingriffselemente auseinandergespreizt werden, insbesondere von der Aussenfläche oder der Aussenumfangsfläche der Nadelabdeckkappe. Allgemein wird das Eingriffselement in etwa radial nach aussen, d. h. von der Längsachse weg von der Nadelschutzkappe ausgelenkt. Während des Verschiebens des Produktbehälters mitsamt der daran befestigten Nadelschutzkappe gleitet die Aussenfläche oder Aussenumfangsfläche der Nadelschutzkappe an dem Eingriffselement entlang. Das Eingriffselement wird am Ende des Verschiebens des Produktbehälters mit einer Bewegung quer zur Längsachse, hin in eine Eingriffsposition bewegt. Das Eingriffselement kann z. B. federnd an dem Entfernerelement angeordnet sein, wodurch es quer zur Längsachse hin in die Eingriffsposition federt. In der Eingriffsposition ist ein Abschnitt der Nadelschutzkappe distal in einer Flucht mit dem Eingriffselement angeordnet. Der Abschnitt ist distal des Eingriffselements angeordnet, wodurch das Eingriffselement gegen das proximale Ende der Nadelschutzkappe drückt oder dort anschlägt, wenn das Entfernerelement zusammen mit der Kappe relativ zu der Nadelschutzkappe in die distale Richtung bewegt wird.

Der Produktbehälterabschnitt der hierin beschriebenen Injektionsvorrichtung kann sich an seinem distalen Ende verjüngen und z. B. in einen Nadelhalteabschnitt übergehen. Zwischen dem sich verjüngenden Ende und dem proximalen Ende der Nadelschutzkappe kann beispielsweise ein Spalt gebildet sein, in dem das Eingriffselement am Ende des Verschiebens des Produktbehälters mit einer Bewegung quer zur Längsachse und zu der Längsachse hin in die Eingriffsposition bewegt wird oder federt.

Die Injektionsvorrichtung umfasst einen Blockierabschnitt, wobei sich der Blockierabschnitt in der Freigabeposition befindet, insbesondere in die proximale Richtung versetzt zu dem Eingriffselement, wenn der Produktbehälter mitsamt der daran befestigten Nadelschutzkappe in dem Aufnahmegehäuse entlang der Längsachse in die distale Richtung verschoben wird. Dadurch kann vorteilhaft erreicht werden, dass die Befestigung des mindestens einen Eingriffselements an dem Entfernerelement so ausgestaltet sein kann, dass nur ein geringer Widerstand gegen ein Auslenken quer nach außen, d. h. in etwa radial von der Längsachse weg des Eingriffselements vorhanden sein kann, wodurch das Auslenken des oder der Eingriffselemente durch die Nadelschutzkappe keine Verschiebung der Nadelschutzkappe an dem Produktbehälter bewirkt, wodurch die Sterilität der Nadel während des Verschiebens des Produktbehälters in dem Aufnahmegehäuse während der Montage der Injektionsvorrichtung nicht gefährdet wird. Insbesondere befindet sich eine gegebenenfalls vorhandene Ausnehmung in Bezug auf die Längsachse auf der Position des Eingriffselements.

Der Blockierabschnitt ist - wie bereits erwähnt - aus der Freigabeposition in die Blockierposition bewegbar. Er befindet sich in der Freigabeposition, wenn die Kappe insbesondere vollständig an dem Aufnahmegehäuse angebracht ist, wie z. B. mit dem Aufnahmegehäuse verschnappt ist und der Produktbehälter mitsamt Nadelschutzkappe in die distale Richtung verschoben ist oder wurde. Der Blockierabschnitt blockiert die Bewegbarkeit des Eingriffselements quer nach außen nicht, wenn der Blockierabschnitt in seiner Freigabeposition ist und blockiert eine Bewegung des Eingriffselements quer nach außen, wenn der Blockierabschnitt in seiner Blockierposition ist.

Der Blockierabschnitt befindet sich auch dann in seiner Freigabeposition, wenn die Injektionsvorrichtung vollständig montiert wurde. Erst zur Vorbereitung einer Injektion, d. h. durch Abziehen oder Entfernen der Kappe von der Injektionsvorrichtung oder deren Gehäuse und nicht schon bei der Montage der Injektionsvorrichtung, wird der Blockierabschnitt aus der Freigabeposition in die distale Richtung in die Blockierposition bewegt.

Beispielsweise kann der Blockierabschnitt von der Kappe oder von dem Entfernerelement gebildet sein. Beispielsweise kann die Kappe eine Ausnehmung aufweisen, die in der Freigabeposition des Blockierabschnitts entlang der Längsachse in eine Position auf Höhe des Eingriffselements ist, wobei der Blockierabschnitt durch Entfernen der Kappe in die Blockierposition bewegt wird. Alternativ kann der von dem Entfernerelement gebildete Blockierabschnitt und das Eingriffselement über einen Verformungsabschnitt des Entfernerelements verbunden sein, der sich während der Bewegung des Blockierabschnitts aus der Freigabeposition in die Blockierposition verformt, insbesondere entlang der Längsachse verlängert.

In Ausführungen, die nicht Teil der Erfindung sind, in denen die Kappe und das Entfernerelement in einem Eingriff sind, der bewirkt, dass ein Abschnitt des Entfernerelements, der über einem Verformungsabschnitt, der die Nadelschutzkappe über ihre Umfangsfläche umgibt, mit dem Eingriffselement verbunden ist, beim Entfernen der Kappe von dem Aufnahmegehäuse von der Kappe mitgenommen wird, wird der Abschnitt, insbesondere der Koppelabschnitt relativ zu dem Eingriffselement bewegt und der Verformungsabschnitt verformt. Dadurch wird der Verformungsabschnitt gegen die Umfangsfläche der Nadelschutzkappe eingeschnürt. Hierdurch kann die Reibung zwischen der Nadelschutzkappe und dem Verformungsabschnitt erhöht und/oder ein Formschluss zwischen der Nadelschutzkappe und dem Verformungsabschnitt hergestellt werden. Zum Beispiel kann der Verformungsabschnitt eine oder mehrere Eingriffsnocken aufweisen, die durch das Einschnüren des Verformungsabschnitts in die Umfangsfläche der Nadelschutzkappe eindringen.

Die Erfindung wurde anhand mehrerer Ausführungsformen und Beispiele beschrieben. Im Folgenden werden besonders bevorzugte Ausführungen der Erfindung anhand von Figuren beschrieben. Die dabei offenbarten Merkmale bilden die Erfindung einzeln und in jeglicher Merkmalskombination vorteilhaft weiter. Es zeigen:
- Figur 1: eine Schnittansicht einer Kappe mit einem darin angeordneten Entfernerelement für eine erste Ausführungsform der Erfindung,
- Figur 2: das Entfernerelement aus Figur 1,
- Figuren 3a und 3b: Schnittdarstellungen eines Aufnahmegehäuses für eine Injektionsvorrichtung mit der Kappe nach Figur 1 und einen teilweise eingesetzten Produktbehälter, wobei Figur 3b eine gegenüber Figur 3a um die Längsachse um 90° gedrehte Ansicht ist,
- Figuren 4a und 4b: die Baugruppe aus den Figuren 3a und 30 während des Verschiebens des Produktbehälters in Bezug auf das Aufnahmegehäuse in die distale Richtung vor dem Erreichen der distalen Endposition, wobei Figur 4b eine gegenüber Figur 4a um die Längsachse um 90° gedrehte Ansicht ist,
- Figuren 5a und 5b: Schnittdarstellungen einer Injektionsvorrichtung mit der Baugruppe aus den Figuren 4a und 4b, wobei sich der Produktbehälter in Bezug auf das Aufnahmegehäuse in einer distalen Endposition befindet, wobei Figur 5b eine gegenüber Figur 5a um die Längsachse um 90° gedrehte Ansicht ist,
- Figuren 6a und 6b: die Injektionsvorrichtung aus den Figuren 5a und 5b, wobei die Kappe von dem Aufnahmegehäuse gelöst und um einen ersten Teilhub in die distale Richtung verschoben wurde, wobei Figur 6b eine gegenüber Figur 6a um die Längsachse um 90° gedrehte Ansicht ist,
- Figuren 7a und 7b: die Injektionsvorrichtung aus den Figuren 6a und 6b, wobei die Kappe mitsamt Nadelschutzhülse von dem Aufnahmegehäuse bzw. die Nadelschutzhülse von dem Produktbehälter entfernt wurde, wobei Figur 7b eine gegenüber Figur 7a um die Längsachse um 90° gedrehte Ansicht ist,
- Figur 8: verschiedene Ansichten eines Entfernerelements für eine zweite Ausführungsform, welche nicht Teil der Erfindung ist,
- Figur 9: die Ansichten aus Figur 8, wobei ein Verformungsabschnitt des Entfernerelements entlang der Längsachse gestreckt und somit vom Durchmesser her eingeschnürt ist,
- Figuren 10a und 10b: Schnittdarstellungen eines Aufnahmegehäuses für eine Injektionsvorrichtung mit einer am distalen Ende des Aufnahmegehäuses befestigten Kappe, in der das Entfernerelement aus Figur 8 angeordnet ist, wobei in dem Aufnahmegehäuse ein Produktbehälter teilweise eingefügt ist, wobei Figur 10b eine in Bezug auf Figur 10a um die Längsachse um 90° gedrehte Ansicht ist,
- Figuren 11a und 11b: Schnittdarstellungen einer Injektionsvorrichtung mit der Baugruppe aus den Figuren 10a und 10b, wobei der Produktbehälter in Bezug auf das Aufnahmegehäuse in eine distale Endposition verschoben ist und am proximalen Ende des Aufnahmegehäuses ein Antriebsgehäuse befestigt ist, wobei Figur 11b eine gegenüber Figur 11a um die Längsachse um 90° gedrehte Ansicht ist,
- Figuren 12a und 12b: die Injektionsvorrichtung aus den Figuren 11a und 11b, wobei die Kappe von dem Aufnahmegehäuse gelöst ist und in Bezug auf das Aufnahmegehäuse um einen ersten Teilhub verschoben wurde, wodurch sich der Verformungsabschnitt wie in Figur 9 dargestellt eingeschnürt hat, wobei Figur 12b eine gegenüber Figur 12a um die Längsachse um 90° gedrehte Ansicht ist,
- Figuren 13a und 13b: die Injektionsvorrichtung aus den Figuren 12a und 12b, wobei die Kappe mitsamt Nadelschutzhülse von dem Aufnahmegehäuse entfernt wurde, wobei Figur 13b eine gegenüber Figur 13a um die Längsachse um 90° gedrehte Ansicht ist,
- Figur 14: verschiedene Ansichten eines Entfernerelements für eine dritte Ausführungsform der Erfindung,
- Figur 15: die Ansichten aus Figur 14, wobei ein Verformungsabschnitt der Entfernerhülse entlang der Längsachse gestreckt und ein Blockierabschnitt in eine Blockierposition verschoben wurde,
- Figuren 16a und 16b: Schnittdarstellungen einer Baugruppe mit einem Aufnahmegehäuse, an dessen distalen Ende eine Kappe befestigt ist, die die Entfernerhülse nach Figur 14 aufnimmt, wobei ein Produktbehälter teilweise in das Aufnahmegehäuse eingefügt ist, wobei Figur 16b eine in Bezug auf Figur 16a um die Längsachse um 90° gedrehte Ansicht ist,
- Figur 17: eine Schnittdarstellung einer Injektionsvorrichtung mit der Baugruppe aus Figur 16a, wobei die Kappe von dem Aufnahmegehäuse gelöst ist und um einen ersten Teilhub entlang der Längsachse in die distale Richtung verschoben wurde,
- Figur 18: die Injektionsvorrichtung aus Figur 17, wobei die Kappe mitsamt Nadelschutzhülse von dem Aufnahmegehäuse entfernt wurde.
- Figur 19: eine Explosionsdarstellung eines Autoinjektors

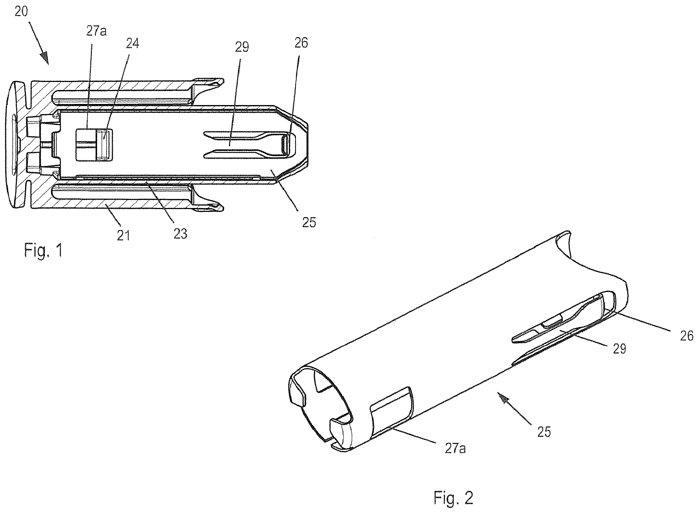

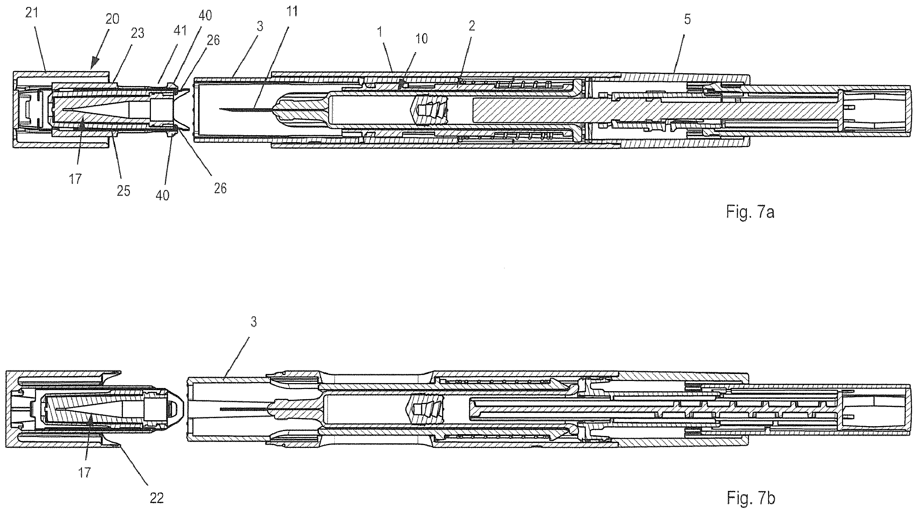

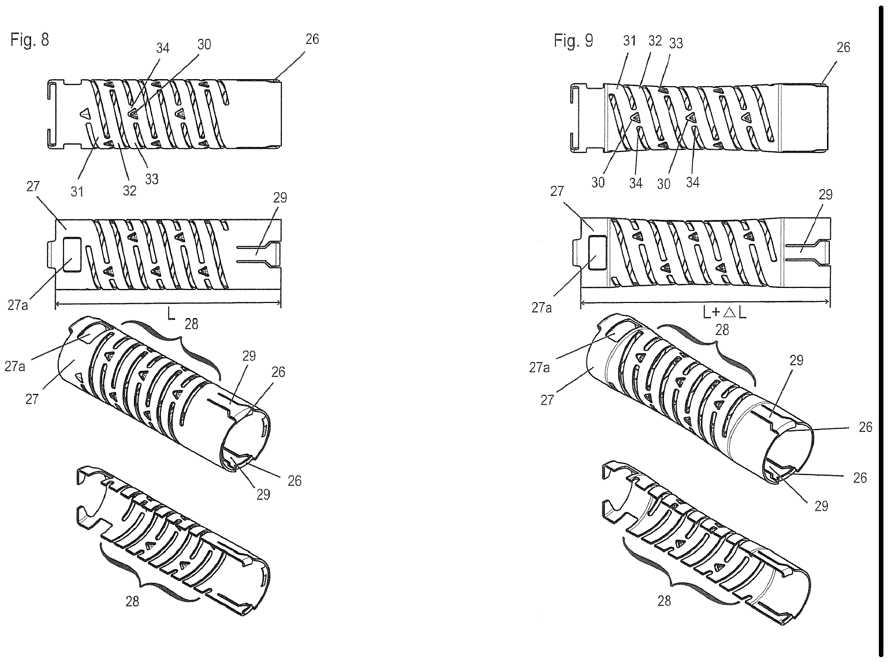

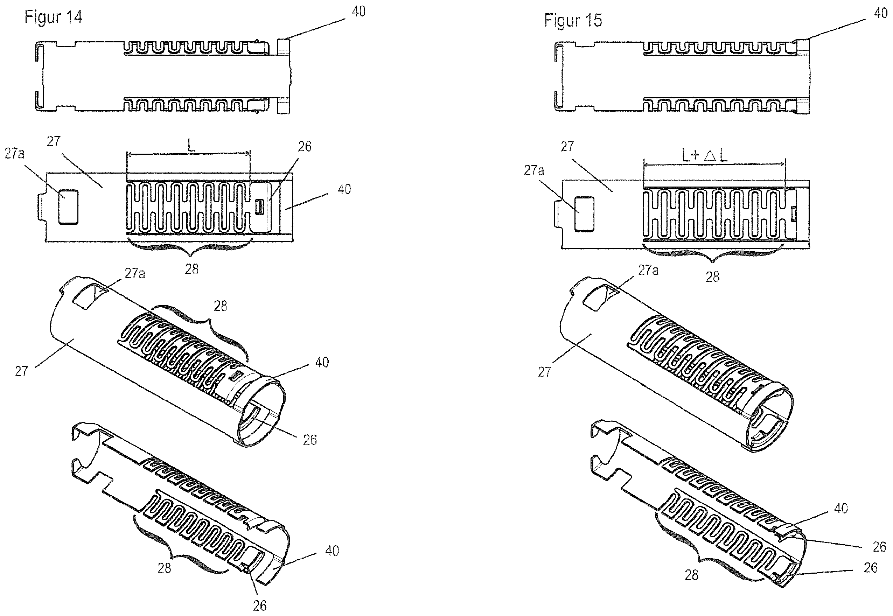

Die erste Ausführungsform, die in den Figuren 1 bis 7b dargestellt wird, die zweite Ausführungsform, die nicht Teil der Erfindung ist, die in den Figuren 8 bis 13b dargestellt wird und die dritte Ausführungsform, die in den Figuren 14 bis 18 dargestellt wird, haben insbesondere den Vorgang gemeinsam, mit dem ein Produktbehälter 10 in die Injektionsvorrichtung eingesetzt wird. Die Injektionsvorrichtung weist ein hufeisenförmiges, insbesondere zylindrisches, Aufnahmegehäuse 1 mit einem proximalen Ende und einem distalen Ende auf. An dem distalen Ende des Aufnahmegehäuses 1 ist eine Kappe 20 angeordnet und an dem Aufnahmegehäuse 1 befestigt. Die Kappe 20 weist einen Hülsenabschnitt 21 auf, der sich wie das Aufnahmegehäuse 1 um eine Längsachse L der Injektionsvorrichtung oder des Aufnahmegehäuses 1 erstreckt. Das distale Ende des Hülsenabschnitts 21 ist im Wesentlichen verschlossen, so dass ein Zugriff von aussen in das Innere der Kappe 20 nicht oder nur erschwert möglich ist. Die Kappe 20 ist mit dem Aufnahmegehäuse 1 formschlüssig verbunden, insbesondere verschnappt. Die Kappe 20 weist an ihrem proximalen Ende ein Eingriffsglied 22 auf, welches in ein entsprechendes Eingriffsgegenglied des Aufnahmegehäuses 1 eingreift, insbesondere damit verschnappt ist. Der formschlüssige Eingriff kann durch eine Bewegung der Kappe 20 um und/oder entlang der Längsachse L gelöst werden, wodurch die Kappe 20 mit einer Bewegung entlang der Längsachse L von dem Aufnahmegehäuse 1 entfernt werden kann.

In der Kappe 20 ist ein vorzugsweise hülsenförmiges Entfernerelement 25, die auch als Entfernerhülse 25 bezeichnet werden oder ausgestaltet sein kann, in Bezug auf die Kappe 20 entlang der Längsachse L - je nach Ausführungsform - verschiebbar oder unverschiebbar aufgenommen.

An dem proximalen Ende des Aufnahmegehäuses 1 kann eine Antriebseinheit, die ein Antriebsgehäuse 5 aufweist, befestigt sein oder werden. Das Antriebsgehäuse 5 kann z. B. kraft- und/oder form- und/oder stoffschlüssig mit dem Aufnahmegehäuse 1 gefügt sein. Das Antriebsgehäuse 5 kann z. B. eine Kolbenstange umgeben, die beispielsweise Bestandteil der Antriebseinheit sein kann. Durch Verschieben der Kolbenstange in die distale Richtung wird ein Produkt aus einem Produktbehälter 10 ausgegeben.

In dem Aufnahmegehäuse 1 ist ein als Spritze ausgestalteter Produktbehälter 10 angeordnet. Der Produktbehälter 10 weist einen Produktbehälterabschnitt 12 auf, der insbesondere hohlzylindrisch gebildet ist und dessen Innenwand mit einem verschiebbar in dem Produktbehälterabschnitt 12 aufgenommenen Kolben 13 einen Dichtspalt bildet. An dem proximalen Ende des Produktbehälterabschnitts 12 kann optional ein Flansch 16, der auch als Fingerflansch bezeichnet wird, angeordnet sein. Der Produktbehälterabschnitt 12 verjüngt sich an seinem distalen Ende zu einem Nadelhalteabschnitt 14 hin, der einen deutlich geringeren Außendurchmesser als der Produktbehälterabschnitt 12 aufweist. Der Nadelhalteabschnitt 14 umgibt den proximalen Teil einer Injektionsnadel 11 und ist damit vorzugsweise unlösbar verbunden. Die Injektionsnadel 11 ragt von dem Nadelhalteabschnitt 14 in die distale Richtung ab. Durch Verschieben des Kolbens 13 in die distale Richtung kann das zwischen der Injektionsnadel 11 und dem Kolben 13 im Produktbehälterabschnitt 12 angeordnete vorzugsweise flüssige Produkt durch die Injektionsnadel 11 hindurch ausgegeben werden.

An dem Nadelhalteabschnitt 14 ist eine Nadelschutzkappe 17 lösbar, wie z. B. form- und/oder reibschlüssig, befestigt. Die Nadelschutzkappe 17 ein ein so genanntes rigid needle shield oder alternativ ein soft needle shield sein. Die Nadelschutzkappe 17 umgibt die Injektionsnadel 11 derart, dass deren Sterilität in Bezug auf die Umgebung der Nadelschutzkappe 17 gewährleistet ist.

Der Produktbehälter 10 kann optional in einem z. B. hülsenförmigen Produktbehälterhalter 2 angeordnet und davon eng eingefasst sein. Beispielsweise kann sich der verjüngende Abschnitt des Produktbehälterabschnitts 12 an einer nach innen ragenden Schulter des Produktbehälterhalters 2 in die distale Richtung abstützen. Alternativ kann sich der Flansch 16 an dem Produktbehälterhalter 2 in die distale Richtung abstützen. Noch weiter alternativ kann der Produktbehälterhalter 2 den Produktbehälter 10 an seinem Produktbehälterabschnitt 12 reibschlüssig halten. Der Produktbehälterhalter 2 kann z.B. axial fest oder verschiebbar in dem Aufnahmegehäuse 1 angeordnet sein.

In dem Aufnahmegehäuse 1 kann optional eine Nadelschutzhülse 3 angeordnet sein, die in Bezug auf das Aufnahmegehäuse 1 zum Auslösen einer Produktausschüttung in die proximale Richtung verschiebbar ist und nach erfolgter Produktausschüttung in die distale Richtung verschiebbar ist, um die Spitze der Injektionsnadel 11 abzudecken, um eine Verletzungsgefahr zu verringern. Solche Nadelschutzhülsen 3 sind aus dem Stand der Technik allerdings bekannt und können als vorteilhafte Weiterbildung der Erfindung angesehen werden.

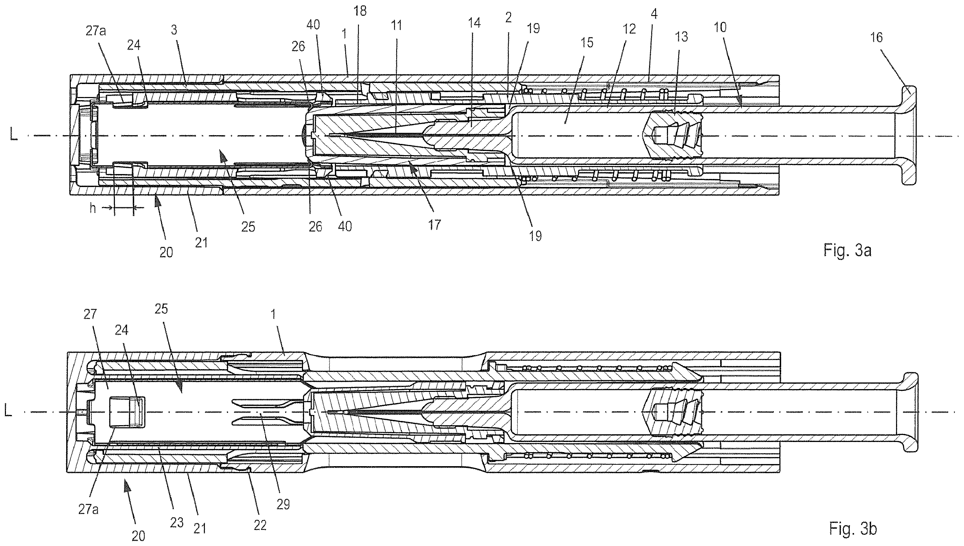

Gemäß der in den Figuren 1 bis 7b dargestellten ersten Ausführungsform nimmt die Kappe 20 das hülsenförmige Entfernerelement 25 auf. Die Kappe 20 und ein Koppelabschnitt 27 des Entfernerelements 25 greifen so ineinander, dass die Kappe 20 in Bezug auf den Koppelabschnitt 27 begrenzt, insbesondere um einen Hub h (Figur 3a) entlang der Längsachse L in die distale Richtung verschiebbar ist. Das Entfernerelement 25 bildet insbesondere einen Axialanschlag, an den ein Teil der Kappe 20 am Ende des Hubs h anschlägt und dadurch bewirkt, dass das Entfernerelement 25 von der Kappe 20 mitgenommen wird, wenn die Kappe 20 entlang der Längsachse L und relativ zu dem Aufnahmegehäuse 1 in die distale Richtung bewegt wird. Der Hub h kann auch als erster Teilhub beispielweise eines für das Entfernen der Kappe 20 von dem Aufnahmegehäuse 1 erforderlichen Gesamthubs bezeichnet werden.

Der Koppelabschnitt 27 oder allgemein das Entfernerelement 25, weist eine Ausnehmung 27a auf, in welche eine Abragung 24, die von der Kappe 20 gebildet wird, eingreift. Das distale Ende der Ausnehmung 27a bildet den Axialanschlag für die Abragung 24, wobei die Abragung 24 an dem distalen Ende der Ausnehmung 27a am Ende des ersten Teilhubs h anstößt. Im vollständig aufgesetzten Zustand der Kappe 20 auf dem Aufnahmegehäuse, d. h., wenn das Aufnahmegehäuse 1 mit der Kappe 20 verschnappt ist, beträgt der Abstand zwischen dem distalen Ende der Ausnehmung 27a und der Abragung 24 in etwa der Länge des ersten Teilhubs h (Figur 3a).

Die Kappe 20 weist eine Innenhülse 23 auf, die z. B. in etwa konzentrisch zu und in dem Hülsenabschnitt 21 angeordnet ist und das Entfernerelement 25 an seinem Außenumfang einfasst oder eng umgibt. Dadurch kann das Entfernerelement 25 von der Kappe 20 entlang der Längsachse L verschiebbar geführt sein. Durch die Führung des Entfernerelements 25 durch die Innenhülse 23 kann ein seitliches Wegkippen des Entfernerelements 25 sicher verhindert werden.

Das Entfernerelement 25 weist ein Eingriffselement 26 auf, welches über einen Arm, insbesondere federnden Arm mit dem Koppelabschnitt 27 verbunden ist. Der Arm 29 ist so ausgestaltet, dass er eine Bewegung des Eingriffselements 26 quer, insbesondere in etwa radial zur Längsachse L ermöglicht. Insbesondere kann das Eingriffselement 26 radial nach außen, d. h. von der Längsachse L weg bewegt werden und in seine ursprüngliche Position zurück federn, d. h. zur Längsachse L hin.

Das Entfernerelement 25 weist ein z. B. hakenförmiges oder nach innen ragendes Eingriffselement 26 auf, welches insbesondere im vollständig montierten Zustand der Injektionsvorrichtung in einer Eingriffsposition in Bezug auf die Nadelschutzkappe 17 ist, wobei in der Eingriffsposition ein Abschnitt der Nadelschutzkappe 17 distal in einer Flucht mit dem Eingriffselement 26 angeordnet ist. In den gezeigten Beispielen greift das Eingriffselement 26 in einen Spalt 19 ein, der zwischen dem proximalen Ende der Nadelschutzkappe 17 und dem sich verjüngenden Übergang von dem Produktbehälterabschnitt 12 in den Nadelhalteabschnitt 14 befindet. Alternativ könnte das Eingriffselement 26 im vollständig montierten Zustand der Injektionsvorrichtung in seiner Eingriffsposition z. B. in eine Ausnehmung am Außenumfang der Nadelschutzkappe eingreifen oder unter Verformung der Nadelschutzkappe 17 in den Außenumfang der Nadelschutzkappe 17 eingreifen.

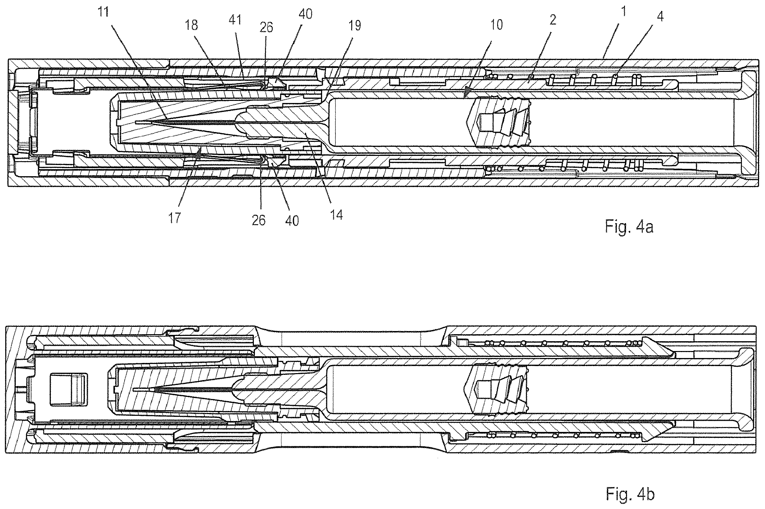

Die Figuren 4a und 4b zeigen die Position des Eingriffselements 26 während des Einschiebens des Produktbehälters 10 in die distale Richtung vor dem Erreichen der distalen Endposition.

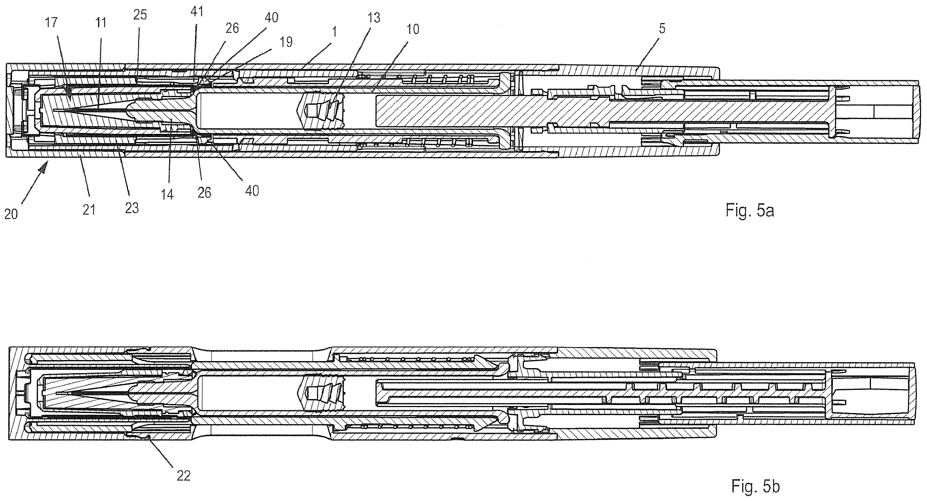

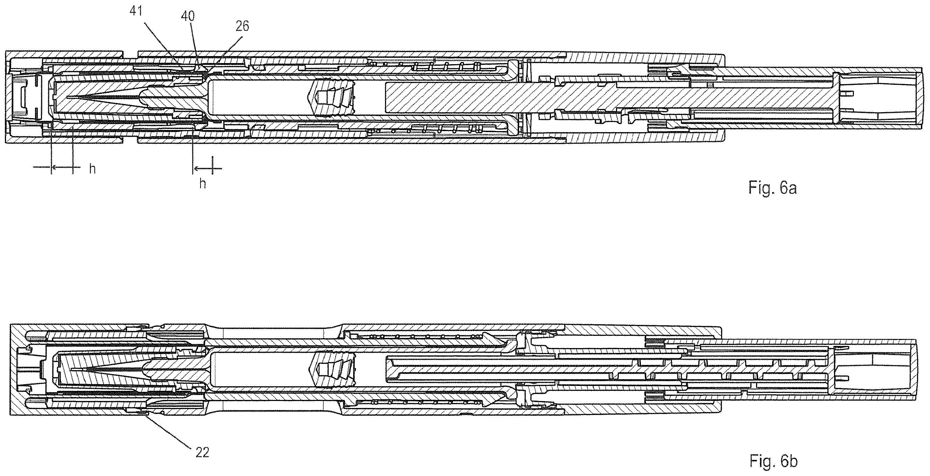

In den Figuren 5a und 5b wird die Injektionsvorrichtung im vollständig montierten Zustand gezeigt, wobei sich das Eingriffselement 26 in der Eingriffsposition befindet. Die Kappe 20 weist einen Blockierabschnitt 40 auf, der in einer Freigabeposition proximal des Eingriffselements 26 angeordnet ist. Beim Abziehen der Kappe 20 wird das Eingriffsglied 22 von dem entsprechenden Eingriffsgegenglied des Aufnahmegehäuses 1 gelöst und die Kappe 20 entlang der Längsachse L in die distale Richtung bewegt. Dabei führt die Kappe 20 entlang der Längsachse L einen ersten Teilhub h in die distale Richtung aus, wodurch der Blockierabschnitt 40 ebenfalls relativ zu dem Eingriffselement 26 um den ersten Teilhub h in die distale Richtung bewegt wird (Figuren 6a, 6b). Am Ende des ersten Teilhubs h stößt die Kappe 20, insbesondere deren Abragung 24 an dem Axialanschlag des Entfernerelements 25, insbesondere dem distalen Ende der Ausnehmung 27a an und befindet sich der Blockierabschnitt 40 in seiner Blockierposition, nämlich in einer Position, in der er die Bewegung des Eingriffselements 26 quer nach außen oder von der Längsachse L weg blockiert (Figuren 6a, 6b). Durch weiteres Bewegen der Kappe 20 entlang der Längsachse L in die distale Richtung wird das Entfernerelement 25 und somit auch das Eingriffselement 26 und die Nadelschutzkappe 17, z.B. während eines zweiten Teilhubs, mitgenommen, wodurch die Nadelschutzkappe 17 von dem Produktbehälter 10, insbesondere dem Nadelhalteabschnitt 14, entfernt wird. Somit wird die Nadelschutzkappe 17 zusammen mit der Kappe 20 von der Injektionsvorrichtung oder dem Aufnahmegehäuse 1 entfernt, wodurch die Injektionsnadel 11 für eine nachfolgende Injektion freigelegt wird. Durch die Positionierung des Blockierabschnitts 40 in der Blockierposition wird verhindert, dass das Eingriffselement 26 während des Abziehens der Kappe 20 vom Aufnahmegehäuse 1 aus einem Eingriff mit der Nadelschutzkappe 17 gerät. Dadurch wird sichergestellt, dass die Nadelschutzkappe 17 immer zusammen mit der Kappe 20 abgezogen wird.

Durch den Eingriff der Abragung 24 in die Ausnehmung 27a ist das Entfernerelement 25 um die Längsachse L verdrehgesichert mit der Kappe 20 verbunden.

Die in den Figuren 14 bis 18 beschriebene dritte Ausführungsform weist das hülsenförmige Entfernerelement 25 auf, welches mit seinem Koppelabschnitt 27 axial fest, d. h. entlang der Längsachse L nicht verschiebbar mit der Kappe 20 verbunden ist. Die Kappe 20 weist eine Abragung 24 auf, welche in die Ausnehmung 27a des Koppelabschnitts 27 eingreift. Das Entfernerelement 25 ist um die Längsachse L verdrehgesichert mit der Kappe 20 verbunden, insbesondere mittels des Eingriffs der Abragung 24 in die Ausnehmung 27a.

Das Eingriffselement 26 ist über einen Verformungsabschnitt 28 mit dem Koppelabschnitt 27 verbunden. Der Verformungsabschnitt 28 ist so ausgestaltet, dass er sich entlang der Längsachse L und insbesondere auch quer zur Längsachse L verformen kann. Insbesondere kann er sich so verformen, dass seine sich entlang der Längsachse L erstreckende Länge I sich zumindest um den Betrag ΔI verlängern kann.

Der Verformungsabschnitt 28 kann einen oder mehrere wendelförmige oder sich mäanderförmig entlang der Längsachse L windende Abschnitte oder Stege aufweisen, die den Koppelabschnitt 27 und das Eingriffselement 26 verbinden. Alternativ oder zusätzlich kann der Verformungsabschnitt 28 eine Vielzahl von entlang der Längsachse L aneinandergereihten ringförmigen Elementen aufweisen, wobei benachbarte ringförmige Elemente über einen Verbindungssteg verbunden sind. Die Ringachse, um die sich die ringe erstrecken steht quer, insbesondere im Wesentlichen senkrecht auf die Längsachse L. Insbesondere können die Ringe so entlang der Längsachse L abgeflacht sein, dass jeder der Ringe einen schlitzförmigen Hohlraum einfasst, wobei sich die Schlitzbreite entlang der Längsachse L erstreckt. Durch eine solche Anordnung wird in einer mechanischen Hinsicht eine Vielzahl von idealisierten Biegebalken gebildet, die sich biegen und somit eine leichte Verformung erlauben, wenn die Länge I vergrößert wird.

Das Entfernerelement 25 weist ferner den Blockierabschnitt 40 auf, der im vollständig montierten Zustand der Injektionsvorrichtung proximal des Eingriffselements 26 angeordnet ist. Das Eingriffselement 26 weist einen Axialanschlag 42 auf, der in dem gezeigten Beispiel aus den Figuren 14 und 15 als eine z. B. durch Umformen oder Stanzbiegung hergestellte Nase ausgebildet ist, die nach außen, d. h. von der Längsachse L weg ragt. Der Axialanschlag 42 befindet sich entlang der Längsachse L in einer Flucht mit dem Blockierabschnitt 40.

Der Blockierabschnitt 40 ist entlang der Längsachse L unverschiebbar mit dem Koppelabschnitt 27 verbunden, wie z. B. über einen Verbindungssteg. In dem gezeigten Beispiel sind zwei längliche Verbindungsstege vorhanden, welche den Koppelabschnitt 27 mit dem Blockierabschnitt 40 verbinden. Der Blockierabschnitt 40 verbindet die beiden Verbindungsstege umfangsseitig, wobei auf der gegenüberliegenden Seite ebenfalls ein weiterer Blockierabschnitt 40 angeordnet ist, der die Verbindungsstege umfangsseitig verbindet. In Umfangsrichtung zwischen den Verbindungsstegen ist der Verformungsabschnitt 28 angeordnet. Gegenüberliegend ist ein weiterer im Wesentlichen baugleicher Verformungsabschnitt 28 angeordnet.

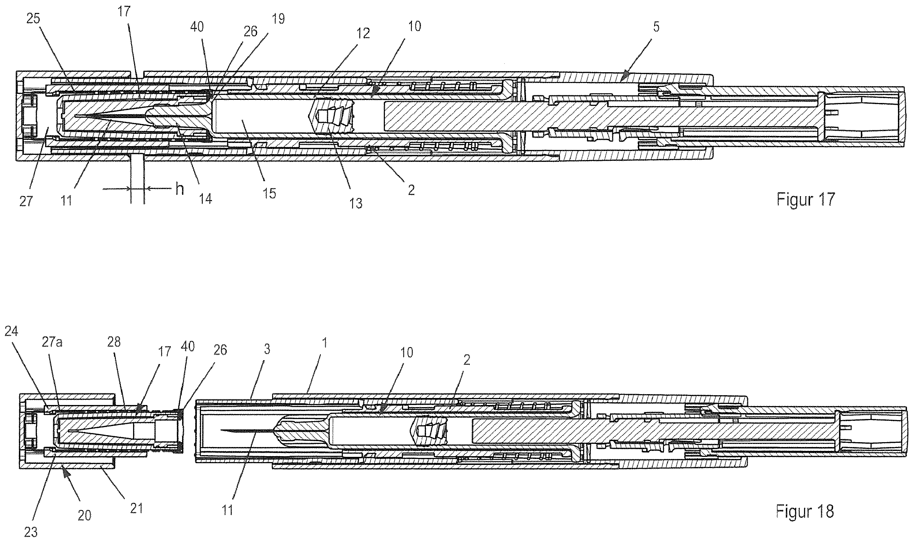

Zum Entfernen der Kappe 20 wird diese aus dem formschlüssigen Eingriff mit dem Aufnahmegehäuse 1 gelöst und zunächst um den ersten Teilhub h entlang der Längsachse in die distale Richtung bewegt. Dadurch, dass der Koppelabschnitt 27 axial fest mit der Kappe 20 verbunden ist, wird der Koppelabschnitt 27 und der damit unverschiebbar verbundene Blockierabschnitt 40 während des ersten Teilhubs h von der Kappe 20 mitgenommen, während das Eingriffselement 26 aufgrund des Eingriffs mit der Nadelschutzkappe 17 relativ zu dem Aufnahmegehäuse 1 oder dem Produktbehälter 10 stillsteht. Dadurch wird die Kappe 20 und somit auch der Koppelabschnitt 27 relativ zu dem Eingriffselement 26 in die distale Richtung bewegt. Gleichzeitig wird der Blockierabschnitt 40 aus der Freigabeposition in die Blockierposition verschoben (Figur 15), wobei der Blockierabschnitt 40 in der Blockierposition an dem Axialanschlag 42 anschlägt. In der Blockierposition verhindert der Blockierabschnitt 40 eine Bewegung des Eingriffselements 26 quer zur Längsachse L nach außen, d. h. von der Längsachse weg. Während der Bewegung des Blockierabschnitts 40 aus der Freigabeposition in die Blockierposition verlängert sich der Verformungsabschnitt 28 um den Betrag ΔI (Figuren 15 und 17).

Dadurch, dass der Blockierabschnitt 40 an dem Axialanschlag 42 ansteht, nimmt der Blockierabschnitt 40 das Eingriffselement 26 bei einer weiteren Bewegung (zweiter Teilhub) entlang der Längsachse L in die distale Richtung mit, wodurch die Nadelschutzkappe 17 mitgenommen und von dem Produktbehälterhalter 10, insbesondere dem Nadelhalteabschnitt 14 abgezogen wird (Figur 18).

In der zweiten Ausführungsform, wobei die zweite Ausführungsform nicht Teil der Erfindung ist, umfasst die Injektionsvorrichtung ein hülsenfoermiges Entfernerelement 25, welches einen Koppelabschnitt 27 aufweist, der axialfest, vorzugsweise dreh- und axialfest mit der Kappe 20 verbunden ist. Die Kappe 20 weist eine Abragung 24 auf, die in eine Ausnehmung 27a des Koppelabschnitts 27 eingreift und bewirkt, dass der Koppelabschnitt 27 in Bezug auf die Kappe 20 entlang der Längsachse L unverschiebbar ist und vorzugsweise um die Längsachse L nicht verdrehbar ist. Das nach innen ragende Eingriffselement 26 ist über einen Verformungsabschnitt 28 mit dem Koppelabschnitt 27 verbunden. Der Verformungsabschnitt 28 ist so ausgebildet, dass er sich entlang der Längsachse L verlängern kann, wodurch sich die Länge l um den Betrag ▲ l verlängert. Mit der Verlängerung schnürt sich der Verformungsabschnitt 28 ein (Figur 9), so dass dessen Innendurchmesser verringert wird, insbesondere in Bezug auf den Innendurchmesser, wenn der Verformungsabschnitt 28 seine ursprüngliche Länge l (Figur 8) aufweist. Der Verformungsabschnitt 28 weist mindestens eine Wendel 31, 32, 33 auf, die sich helixförmig mit mindestens einer vollständigen Windung um die Längsachse L windet und den Koppelabschnitt 27 mit einem Basisabschnitt 43 verbindet. Das Eingriffselement 26 ist über einen Arm 29 mit dem Basisabschnitt 43 so verbunden, dass das Eingriffselement 26 quer zur Längsachse L bewegbar, insbesondere federnd bewegbar ist. Der Verformungsabschnitt 28 weist in dem gezeigten Beispiel mehrere Wendeln 31, 32, 33, wie z. B. eine erste Wendel 31, eine zweite Wendel 32 und eine dritte Wendel 33 auf, die sich helixförmig jeweils mit mindestens einer Windung, hier sogar mehr als zwei Windungen, um die Längsachse L erstrecken und den Koppelabschnitt 27 mit dem Basisabschnitt 43 verbinden. Benachbarte Windungen der Wendeln 31, 32, 33 sind über einen Verbindungssteg verbunden. Einer oder mehrere oder alle dieser Verbindungsstege weisen eine Eingriffsnocke 30 auf, welche vom Innenumfang des Verformungsabschnitts 28 nach innen ragt, insbesondere mit einer in die distale Richtung gerichteten Spitze der Eingriffsnocke 30.

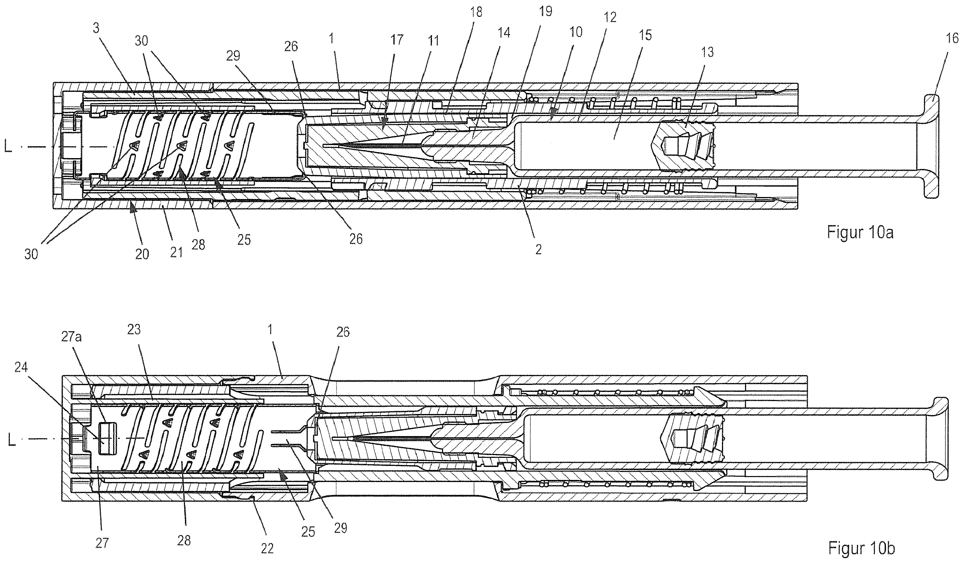

Zum Einsetzen wird der Produktbehälter 10 in das Aufnahmegehäuse 1 eingesetzt (Figuren 10a, 10b) und bis zum Erreichen seiner distalen Endposition in die distale Richtung verschoben, wodurch das Eingriffselement 26 in den Spalt 19 federt und die Eingriffsposition einnimmt.

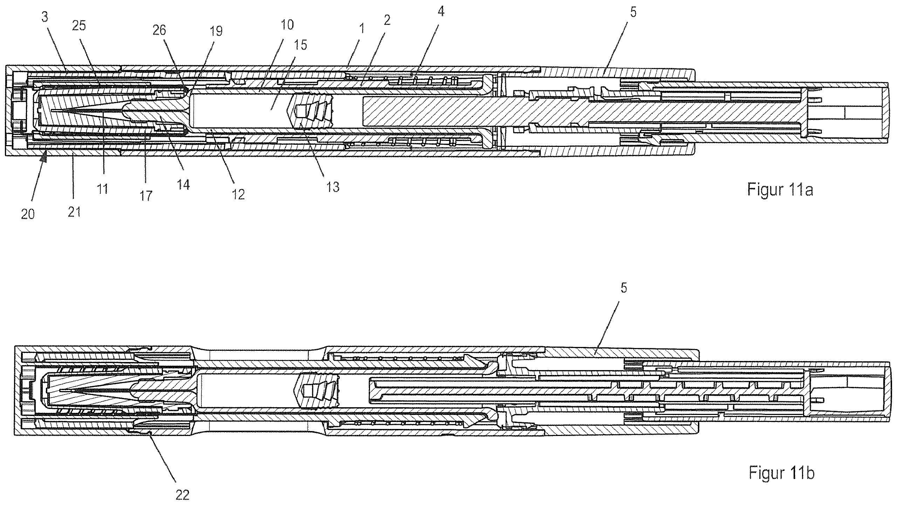

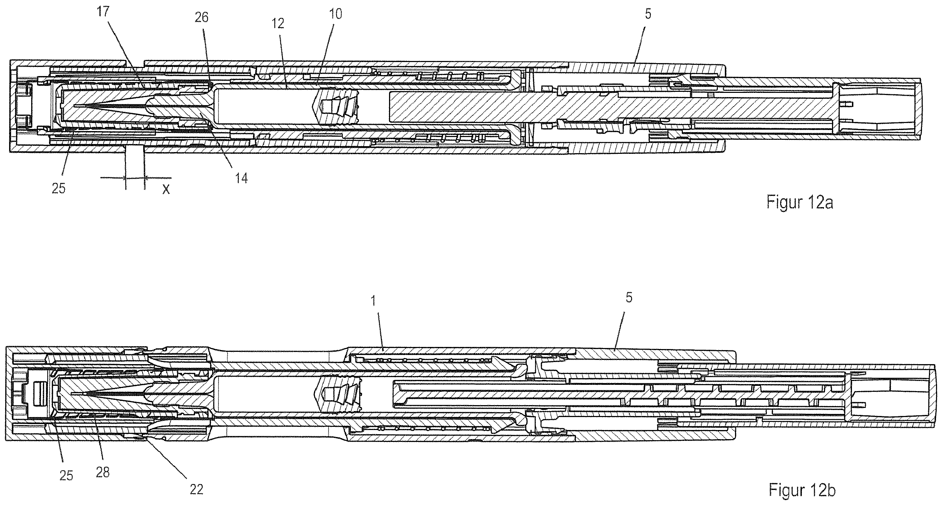

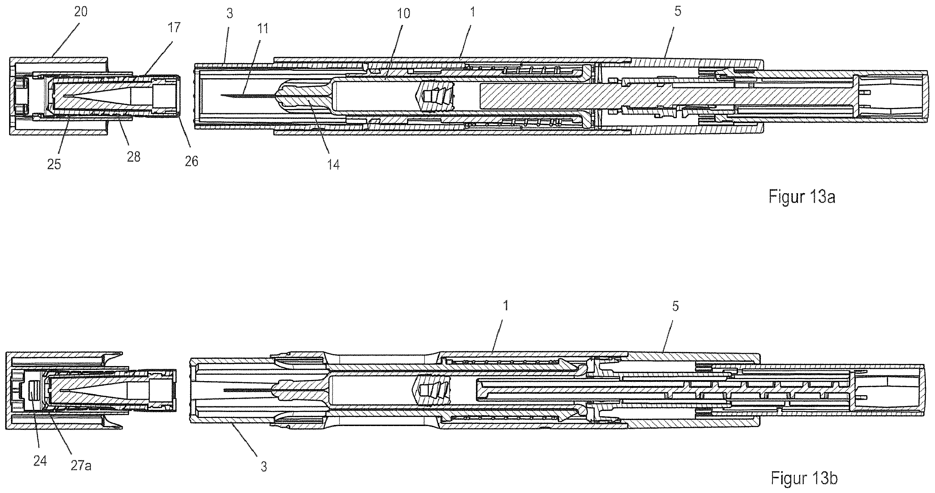

Im vollständig montierten Zustand der Injektionsvorrichtung befindet sich das Eingriffselement 26 in seiner Eingriffsposition (Figuren 11a, 11b). Zum Entfernen der Kappe 20 wird zunächst der formschlüssige Eingriff mit dem Aufnahmegehäuse 1 gelöst und die Kappe 20 um einen Weg oder Teilhub x in die distale Richtung verschoben (Figuren 12a, 12b), wodurch der Koppelabschnitt 27 und die Kappe 20 relativ zu dem Eingriffselement 26 bewegt werden, welches sich an dem proximalen Ende der Nadelschutzkappe 17 in die proximale Richtung abstützt. Hierdurch wird der Verformungsabschnitt 28 um den Betrag Δl (Figur 9) verlängert, wobei sich der Verformungsabschnitt 28 einschnürt und sich die Eingriffsnocken 30 in den Außenumfang oder die Außenumfangsfläche der Nadelschutzkappe 17 einkrallen, wodurch die auf die Kappe 20 ausgeübte Kraft in die distale Richtung nicht nur über das Eingriffselement 26 auf die Nadelschutzkappe 17 übertragen werden, sondern auch über die in die Nadelschutzkappe 17 eingreifenden Eingriffsnocken 30. Wird die Kappe 20 nun weiter in die distale Richtung bewegt, löst sich der Eingriff der Nadelschutzkappe 17 mit dem Produktbehälter, insbesondere dem Nadelhalteabschnitt 14, wodurch die Nadelschutzkappe 17 zusammen mit der Kappe 20 von der Injektionsvorrichtung entfernt wird (Figuren 13a, 13b).

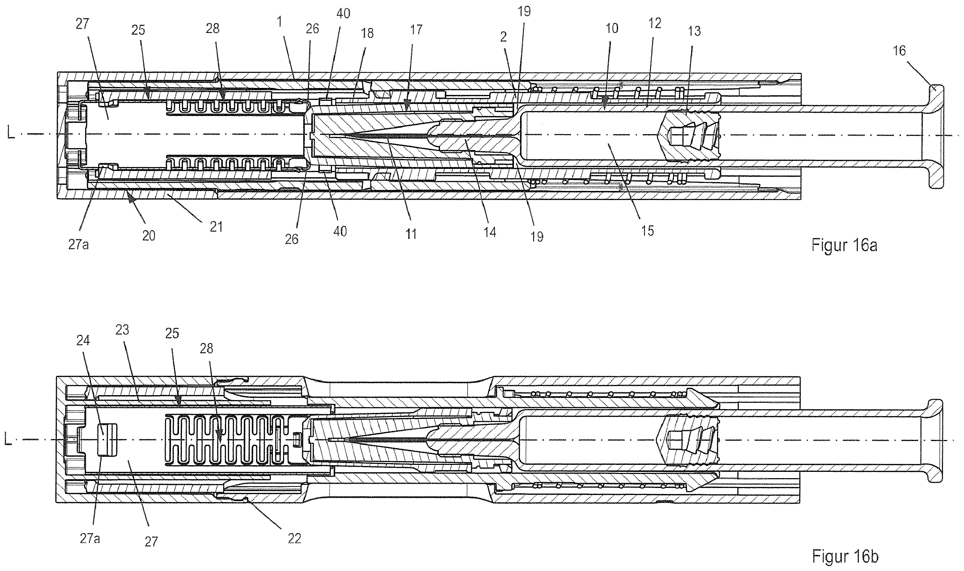

Der Montagevorgang, insbesondere die Integration des Produktbehälters 10 in dem Aufnahmegehäuse 1 der Injektionsvorrichtung, ist in den drei in den Figuren bezeichneten Ausführungsformen im Wesentlichen ähnlich. Der Produktbehälter 10, an dem die Nadelschutzkappe 17 bereits vor dem Einsetzen in das Aufnahmegehäuse 1 befestigt ist, um die Injektionsnadel 11 steril zu halten, wird mit der Nadelschutzkappe 17 voran über eine Öffnung am proximalen Ende des Aufnahmegehäuses 1 entlang der Längsachse L in die distale Richtung in das Aufnahmegehäuse 1 eingeführt. Insbesondere wird der Produktbehälter 10 dabei in den im Aufnahmegehaeuse 1 gelagerten Produktbehaelterhalter 2 eingeführt. Der Produktbehälterhalter 2, der in den gezeigten Beispielen in Bezug auf das Aufnahmegehaeuse 1 verschiebbar ist, stützt sich zum Blockieren seiner Verschiebbarkeit entlang der Längsachse L während der Montage an der Kappe 20 ab, wodurch eine Verschiebung des Produktbehälterhalters 2 in die distale Richtung verhindert wird, wenn die Kappe 20 am Aufnahmegehäuse 1 befestigt ist. Durch das Einschieben des Produktbehälters 10 in das Aufnahmegehäuse 1 gleitet das Eingriffselement 26 an der Nadelschutzkappe 17 ab und wird von der Nadelschutzkappe 17, insbesondere deren Aussenumfangsfläche, radial nach aussen gedrückt. Insbesondere ist dies in der ersten und dritten Ausführungsform deshalb möglich, weil der Blockierabschnitt 40 sich in seiner Freigabeposition und nicht in der Blockierposition befindet (Figuren 4a, 4b, 16a, 16b). In der zweiten Ausführungsform, welche nicht Teil der Erfindung ist, ist zwar kein Blockierabschnitt 40 vorhanden, jedoch verstärkt sich der Eingriff des Entfernerelements 25 mit der Nadelschutzkappe 17 durch das Einschnüren des Verformungsabschnitts 28 beim Abziehen der Kappe 20. Wenn der Produktbehälter 10 vollständig in das Aufnahmegehäuse 1 eingeschoben ist, rastet das Eingriffselement 26 in seine Eingriffsposition zwischen dem sich verjüngenden Ende des Produktbehälterabschnitts 12 und dem proximalen Ende der Nadelschutzkappe 17. Anschliessend wird das Antriebsgehäuse 5 mit dem Aufnahmegehäuse 1 gefügt oder verbunden, wodurch die Injektionsvorrichtung fertig montiert ist.

Dadurch, dass ein Blockierabschnitt 40 vorgesehen ist, der sich beim Abziehen der Kappe 20 in der zweiten Ausführungsform, welche nicht Teil der Erfindung ist, einschnürt und in der ersten und dritten Ausführungsform relativ zum Eingriffselement entweder durch Teilhub oder durch Deformation verschiebt, und dadurch zumindest einen Teil der auf die Kappe 20 aufgebrachten Abziehkraft auf die Nadelschutzkappe 17 überträgt, können in allen Ausführungsformen die jeweiligen Eingriffselemente 26 so an dem Entfernerelement 25 angeordnet sein, dass sie mit einer relativ geringen Kraft nach aussen, d. h. in etwa radial von der Längsachse L weg bewegt oder schwenken können. Hierdurch wird die Gefahr, wie sie z. B. mit einem zu stark ausgebildeten Eingriffselement bestehen würde, verringert, dass die Nadelschutzkappe 17 während des Einführens in das Aufnahmegehäuse von dem Eingriffselement an dem Nadelhalteabschnitt 14 des Produktbehälters 10 bewegt wird. Eine Bewegung der Nadelschutzkappe 17 am Nadelhalteabschnitt 14 kann die Sterilität der Injektionsnadel 11 gefährden.

Im Folgenden wird ein Autoinjektor gezeigt der mit einer Abziehkappe, vorzugsweise aber nicht notwendigerweise einer Abziehkappe der oben beschriebenen Arten, eingesetzt werden kann.

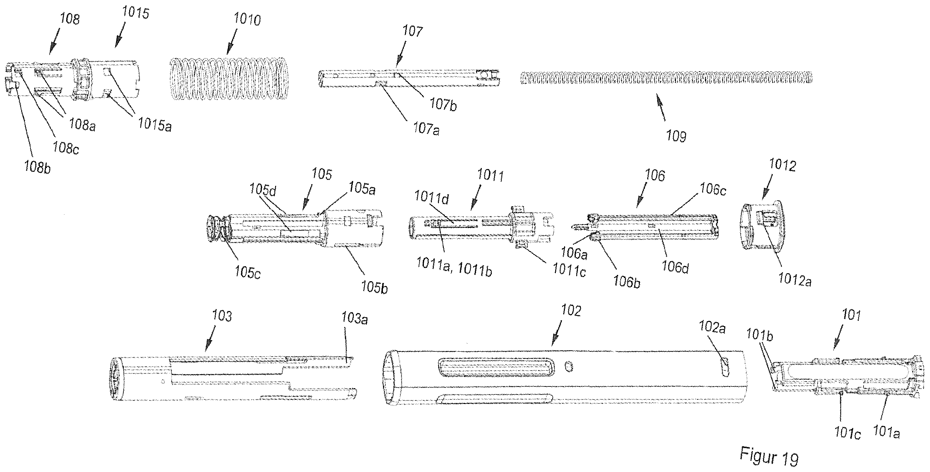

Der Autoinjektor (Figur 19) weist ein hülsenförmiges, längliches Gehäuse 102 mit einer Längsachse L auf, welches an seinem proximalen Ende eine Verschlusskappe 1012 aufweist, die formschlüssig mit dem Gehäuse 102 dreh- und axialfest verbunden ist und das proximale Ende des Autoinjektors bildet. Die Verschlusskappe 1012 ist mit dem Gehäuse 102 verschnappt. Hierfür weist die Verschlusskappe 1012 ein Rastglied 1012a auf, welches in eine Ausnehmung 102a am Gehäuse 102 eingerastet ist, vorzugsweise so, dass die Verschlusskappe 1012 nicht oder nicht ohne weiteres von dem Gehäuse 102 lösbar ist.

An dem distalen Ende des Autoinjektors kann in seinem Auslieferungszustand eine Nadelschutzkappe 20 (in Fig. 19 nicht dargestellt) der oben beschriebenen Arten angeordnet sein, die vor der Verwendung des Autoinjektors abgezogen, oder abgedreht, und entfernt wird.

In dem Gehäuse 102 ist ein Produktbehälter in der Gestalt einer Spritze in Bezug auf das Gehäuse 102 - abgesehen von der Montage des Autoinjektors - entlang der Längsachse L unverschiebbar aufgenommen. Der Produktbehälter weist einen hülsenförmigen Spritzenkörper auf, der einen Kolben, der dichtend an dem Innenumfang des Spritzenkörpers anliegt, umgibt. Der Spritzenkörper weist an seinem distalen Ende eine insbesondere unlösbar mit dem Spritzenkörper verbundene Injektionsnadel auf, deren distales Ende von der Nadelspitze gebildet wird. Zwischen der Injektionsnadel und dem Kolben ist ein flüssiges Produkt, insbesondere Medikament, innerhalb des Spritzenkörpers angeordnet, wobei das flüssige Produkt durch Verschieben des Kolbens in eine Ausschüttrichtung, d. h. in distale Richtung oder zu der Injektionsnadel hin das Produkt durch die hohle Injektionsnadel aus dem Produktbehälter ausschüttet. Der Spritzenkörper weist an seinem proximalen Ende einen sogenannten Fingerflansch auf, der radial nach außen über den Außenumfang des zylindrischen Spritzenkörpers ragt.

Der Produktbehälter ist in einem Produktbehälterhalter, der als Spritzenhalter 101 bezeichnet wird, so aufgenommen, dass er zumindest gegen eine Bewegung entlang der Längsachse L in distale Richtung relativ zu dem Spritzenhalter 101 gesichert ist. Der Spritzenhalter 101 ist, mit dem Gehäuse 102 formschlüssig verbunden, insbesondere verrastet. Das Gehäuse 102 weist hierzu Ausnehmungen auf, in die Rastelemente, die hier am proximalen Ende des Spritzenhalters 101 gebildet werden, eingreifen. Der Spritzenhalter 101 weist mindestens eine nach innen ragende Schulter 101b auf, an der sich ein verjüngender Abschnitt des Produktbehälters, abstützt.

Um zu verhindern, dass der Produktbehälter relativ zu dem Spritzenhalter 101 in die proximale Richtung verschiebbar ist, wird der Produktbehälter an seinem proximalen Ende durch einen auf den Spritzenkörper wirkenden Halter in den Eingriff mit der Schulter 101b gedrückt. Der Halter wird von einem Haltefederabschnitt 105c eines Mechanikhalters 105 gebildet. Der Mechanikhalter 105 ist in Bezug auf das Gehäuse 102 entlang der Längsachse L insbesondere unverschiebbar und/oder drehfest angeordnet. Der hülsenförmige Mechanikhalter 105 kann mit dem Gehäuse 102 verschnappt sein. Durch den Haltefederabschnitt 105c können Längenunterschiede des Produktbehälters, die aufgrund von Herstellungstoleranzen entstehen können, ausgeglichen werden, wobei der feste Sitz des Produktbehälters an der Schulter 101b sichergestellt ist.

Der Produktbehälter ist in Bezug auf das Gehäuse 102 so angeordnet, dass die Nadelspitze distal über das distale Ende des Gehäuses 102 ragt. Im Ausgangs- oder Auslieferungszustand des Autoinjektors, d. h., wenn die Abziehkappe 20 an dem Autoinjektor angeordnet ist, wird die Nadel von einer Nadelabdeckkappe 17, die in den gezeigten Beispielen als sogenanntes und dem Fachmann bekanntes rigid needle shield, alternativ als soft needle shield, ausgebildet ist, abgedeckt, um die Nadel vor Verschmutzung zu schützen bzw. die Nadel 13a und das Medikament steril zu halten. Das rigid needle shield ist an einem Nadelhalteabschnitt des Spritzenkörpers angeordnet, wobei sich der verjüngende Abschnitt des Spritzenkörpers zwischen dem Nadelhalteabschnitt und dem zylindrischen Abschnitt des Spritzenkörpers befindet. Die Schulter 101b ist zwischen dem Spritzenkörper und dem proximalen Ende des rigid needle shield angeordnet, insbesondere so, dass zwischen dem rigid needle shield und der Schulter 101b ein - wenngleich auch geringer - Spalt entsteht, um zu verhindern, dass die Schulter 101b eine Kraft auf das rigid needle shield ausübt, wodurch z. B. die Sterilität der Nadel oder des flüssigen Produkts gefährdet werden könnte. Die Abziehkappe 20 ist mit dem Gehäuse 102 oder einer Nadelschutzhülse 103 lösbar verschnappt, wobei diese Verschnappung gelöst wird, wenn die Abziehkappe 20 von dem Gehäuse 102 oder der Nadelschutzhülse 103 entfernt wird.

Der Autoinjektor weist eine Nadelschutzhülse 103 auf, die relativ zu dem Gehäuse 102 und entlang der Längsachse L um einen Betätigungshub H_{B} in die proximale Richtung in eine betätigte Position verschiebbar ist, um eine Produktausschüttung auszulösen. In der Ausgangsposition der Nadelschutzhülse 103, steht das distale Ende der Nadelschutzhülse 103 distal über die Nadelspitze der Nadel über, so dass ein Zugriff auf die Nadelspitze zunächst verhindert wird. Durch Verschieben der Nadelschutzhülse 103 um den Betätigungshub H_{B} wird die Nadelschutzhülse 103 soweit in die proximale Richtung verschoben, dass die Nadel aus dem distalen Ende der Nadelschutzhülse 103 hervortritt, insbesondere mit einer Länge hervortritt, welche der Injektionstiefe der Nadel in die Einstichstelle entspricht. Bevorzugt soll die Nadel soweit über das distale Ende der Nadelschutzhülse 103 überstehen, dass eine subkutane Injektion erfolgen kann. Insbesondere kann das Gehäuse 102 einen Anschlag bilden, an dem die Nadelschutzhülse 103 in der betätigten Position anliegt.

Nach der erfolgten Injektion kann die Nadelschutzhülse 103 relativ zu dem Gehäuse 102 aus der betätigten Position entlang der Längsachse L um einen Nadelschutzhub H_{N} in die distale Richtung in eine Nadelschutzposition verschoben werden. In der Nadelschutzposition steht das distale Ende der Nadelschutzhülse 103 distal über die Nadelspitze über, so dass ein Zugriff auf die Nadelspitze verhindert und eine Verletzungsgefahr verringert wird. Die Nadelschutzhülse 103 kann, wie weiter unten beschrieben wird, gegen erneutes Zurückschieben aus der Nadelschutzposition blockiert werden.

Der Spitzenhalter 101 weist eine Abragung 101a, die radial nach außen weist, auf, wobei die Abragung 101a in eine schlitzförmige Ausnehmung der Nadelschutzhülse 103, die zwischen dem Gehäuse 102 und dem Spritzenhalter 101 angeordnet ist, eingreift. In der Ausgangsposition der Nadelschutzhülse 103 und/oder in der Nadelschutzposition der Nadelschutzhülse 103 liegt die Nadelschutzhülse 103, insbesondere das proximale Ende der schlitzförmigen Ausnehmung an der Abragung 101a an, wodurch eine Bewegung der Nadelschutzhülse 103 in die distale Richtung verhindert wird. In diese schlitzförmige Ausnehmung, alternativ in eine andere Ausnehmung der Nadelschutzhülse 103, kann ein Nocken 101c, der federnd an dem Spritzenhalter 101 angeordnet und von dem Spritzenhalter 101 gebildet ist, eingreifen. Der Nocken 101c ist so gestaltet, dass bei dem Versuch, die Nadelschutzhülse 103 aus der Ausgangsposition in die betätigte Position zu verschieben, der Nocken 1c die Verschiebung der Nadelschutzhülse 103 zunächst verhindert, wobei der Nocken 101c herausgedrückt wird, wenn die auf die Nadelschutzhülse 103 zum Zurückschieben aufgebrachte Kraft einen gewissen Schwellwert überschreitet, wodurch die Nadelschutzhülse 103 schlagartig in die betätigte Position zurückgeschoben wird. Die Nadel kann hierdurch schlagartig in die Einstichstelle eingestochen werden. Zum Einstechen der Nadel bzw. zum Verschieben der Nadelschutzhülse 103 in die betätigte Position wird das distale Ende der Nadelschutzhülse 103 in die Einstichstelle angesetzt, wobei das Gehäuse 102 dann in Richtung Einstichstelle gedrückt wird, wobei wenn die Andrückkraft den oben genannten Schwellwert überschreitet, das Gehäuse 102 schlagartig zu der Einstichstelle hin und die Nadelschutzhülse 103 relativ zu dem Gehäuse 102 in die betätigte Position verschoben wird.

Das Gehäuse 102 weist einen ringförmigen Halteabschnitt oder Ringabschnitt auf, der insbesondere das distale Ende des Spritzenhalters 101 insbesondere ringförmig umgibt, und daran anliegt, wodurch die mindestens eine Schulter 101b in dem Eingriff mit dem verjüngenden Bereich des Spritzenkörpers gehalten wird. Ferner weist das Gehäuse 102 im Bereich des Halteabschnitts einen Translationsanschlag in der Gestalt einer Halteschulter auf, die verhindert, dass der Spritzenhalter 101 relativ zu dem Gehäuse 102 in die distale Richtung verschiebbar ist, wenn der Spritzenhalter 1 an der Halteschulter anliegt. Dies gilt auch vorteilhaft für die beschriebenen Varianten.

Der Autoinjektor weist ferner ein hülsenförmiges Vortriebsglied 107 auf, welches an seinem distalen Ende eine nach innen ragende Schulter bildet, an der sich eine erste Feder 109, die auch als Ausschüttfeder bezeichnet werden kann, abstützt. Die erste Feder 109 ist innerhalb des hülsenförmigen Vortriebsglieds 107 angeordnet. Die zweite Feder 109 ist eine als Druckfeder wirkende Wendelfeder, die im Ausgangs- oder Auslieferungszustand des Autoinjektors mit so viel Energie vorgespannt ist, dass sie das in dem Produktbehälter enthaltene Produkt insbesondere vollständig durch Verschieben des Vortriebsglieds 107 um einen Ausschütthub H_{A} aus dem Produktbehälter ausschütten kann. Im Auslieferungszustand der Vorrichtung besteht zwischen dem Kolben und dem distalen Ende des Vortriebsglieds 107 ein Abstand, so dass das Vortriebsglied 107 erst während der Ausführung des Ausschütthubs H_{A} an den Kolben anschlägt und diesen in die Ausschüttrichtung mitnimmt.

Die erste Feder 109 stützt sich mit ihrem proximalen Ende an einem Halteelement 106 ab, welches in diesem Beispiel zwei Arme 106c aufweist, wobei an jedem Arm 106c ein erstes Eingriffselement 106a und ein zweites Eingriffselement 106b angeordnet ist. Das erste Eingriffselement 106a weist radial zu der Längsachse L hin, wobei das zweite Eingriffselement 106b radial von der Längsachse L weg weist. Das erste Eingriffselement 106a greift in eine erste Ausnehmung 107a, die von dem Vortriebselement 107 gebildet wird, ein, wodurch eine Bewegung des Vortriebsglieds 7 relativ zu dem Halteelement 106 in die distale Richtung oder in die Ausschüttrichtung verhindert wird. Hierdurch wird die erste Feder 109 in ihrem gespannten Zustand gehalten. Das Halteelement 6 weist einen Führungsstift 106d auf, der durch das proximale Ende der ersten Feder 109 in die Seele der Feder 109 eingefügt ist. Der Führungsstift 106d verhindert ein seitliches Ausknicken der ersten Feder 109 während und am Ende des Ausschütthubs H_{A} des Vortriebsglieds 107.

Der Autoinjektor weist ein Schaltmodul 108, 1015 auf, welches eine Schalthülse 1015 und eine von der Schalthülse 1015 umgebene Sperrhülse 108 aufweist. Im Auslieferungszustand der Vorrichtung wird das erste Eingriffselement 106a von dem Innenumfang der Sperrhülse10 8, der an dem zweiten Eingriffselement 106b anliegt, in dem Eingriff mit der ersten Ausnehmung 107a gehalten.

Die Schalthülse 1015 ist mit dem proximalen Ende 103a der Nadelschutzhülse 103 verbunden oder liegt zumindest an dem proximalen Ende 103a der Nadelschutzhülse 103 an. Eine zweite Feder 1010, innerhalb der die erste Feder 109 angeordnet ist und die vorzugsweise die Schalthülse 1015 und die Sperrhülse 108 zumindest teilweise umgibt, stützt sich mit ihrem distalen Ende an der Schalthülse 1015 ab. Ein Teil der Schalthülse 1015 ist somit zwischen der Nadelschutzhülse 103 und dem distalen Ende der zweiten Feder 1010 angeordnet. Die zweite Feder 1010 ist eine als Druckfeder wirkende und als Wendelfeder ausgestaltete Feder aus Metall. Die zweite Feder 1010 stützt sich mit ihrem proximalen Ende an einem Signalglied 1011, insbesondere an einer Abragung 1011c, die axial verschiebbar und verdrehfest in das Gehäuse 102 eingreift und die durch eine schlitzförmige Nut 105b des Mechanikhalters 105 hindurchgreift, ab. Die zweite Feder 1010 umgibt somit auch den Mechanikhalter 105 zumindest teilweise, vorzugsweise vollständig.

Das Schaltglied 1015 weist eine Ausnehmung 1015a auf, in welche ein Verriegelungsglied 8a der Sperrhülse 108 eingreift. Das Verriegelungsglied 108a ist sägezahnförmig und ragt radial von der Längsachse L weg. Das Verriegelungsglied 8a ist federnd an einem Arm, welcher von der Sperrhülse 108 gebildet wird, angeordnet. Durch Verschieben der Schalthülse 1015 in die proximale Richtung wird die Sperrhülse 8 über den Eingriff des Verriegelungsglieds 108a in die proximale Richtung mitgenommen.

Durch Verschieben der Nadelschutzhülse 103 in die betätigte Position wird die Schalthülse 1015 ebenfalls um den Betätigungshub H_{B} mitgenommen, wodurch die zweite Feder 1010 gespannt wird. Wird die Nadelschutzhülse 103 nicht vollständig in die betätigte Position verschoben, kann die zweite Feder 1010 die Schalthülse 1015 und die Nadelschutzhülse 103 wieder zurück in die Ausgangsposition verschieben, wobei über den Eingriff des Verriegelungsglieds 108a auch die Sperrhülse 108 von der Schalthülse 1015 mitgenommen wird.

Das insbesondere hülsenförmige Signalglied 1011 ist im Auslieferungszustand oder vor der Auslösung der Produktausschüttung in einem axialfesten Eingriff mit dem Vortriebsglied 107. Das Signalglied 1011 weist ein erstes Eingriffsglied 1011a, welches in eine Ausnehmung 107b des Vortriebsglieds 107 eingreift, und ein zweites Eingriffsglied 1011b auf. Das erste Eingriffsglied 1011a und das zweite Eingriffsglied 1011b sind an dem Ende eines Arms 1011d federnd angeordnet. Das Signalglied 1011 weist zwei solcher Arme 1011d mit einem ersten Eingriffsglied 1011a und einem zweiten Eingriffsglied 1011b auf. Das erste Eingriffsglied 1011a weist radial zu der Längsachse L hin, wobei das zweite Eingriffsglied 1011b radial von der Längsachse L weg weist. Im Auslieferungszustand wird das erste Eingriffsglied 1011a von dem Innenumfang der Sperrhülse 108 in dem axialfesten Eingriff mit dem Vortriebsglied 107 gehalten. Das zweite Eingriffsglied 1011b liegt an dem Innenumfang der Schalthülse 108 an. Die Verschlusskappe 1012 weist einen Signalanschlag 12b auf, an den das Signalglied 1011 zur Erzeugung eines Signals anschlagen kann und vorzugsweise an dem das Signalglied 1011 im Auslieferungszustand der Vorrichtung anliegt.

Zur Verabreichung des Produkts aus dem Produktbehälter wird die Abziehkappe 20 von dem Autoinjektor zusammen mit dem rigid needle shield entfernt. Das distale Ende der Nadelschutzhülse 103 wird an die Einstichstelle eines Patienten angesetzt, wobei das Gehäuse 102 zu der Einstichstelle hin verschoben wird, wodurch sich die Nadelschutzhülse 103 aus ihrer Ausgangsposition um den Betätigungshub H_{B} in die proximale Richtung relativ zu dem Gehäuse 102 in die betätigte Position bewegt. Hierdurch wird die zweite Feder 1010 gespannt, wobei die Schalthülse 1015 von der Nadelschutzhülse 103 um den Betätigungshub H_{B} mitgenommen wird. Die Sperrhülse 108 weist eine erste Ausnehmung 108b auf, die durch Verschieben der Sperrhülse 108 um den Betätigungshub H_{B} entlang der Längsachse L auf die Position des zweiten Eingriffselements 106b gebracht wird. Hierdurch wird das erste Eingriffselement 106a aus dem Eingriff mit dem Vortriebsglied 107 mit einer Bewegung quer zu und von der Längsachse L weg bewegt, wobei gleichzeitig das zweite Eingriffselement 106b in den Eingriff mit der Sperrhülse 108, insbesondere deren erster Ausnehmung 108b bewegt wird. Hierdurch wird das Vortriebsglied 107 für die Bewegung um den Ausschütthub H_{A} in die Ausschüttrichtung freigegeben.

Da die axialfeste Kopplung zwischen dem Vortriebsglied 107 und dem Halteelement 106 nun aufgehoben ist, kann das Halteelement 106, welches zumindest ein Stück weit relativ zu dem Gehäuse 102 und entlang der Längsachse L bewegbar ist, von der ersten Feder 109 in die proximale Richtung bewegt werden, wobei das Halteelement 106 über den Eingriff des zweiten Eingriffselements 106b in die Ausnehmung 108b die Sperrhülse 108 um einen Startsignalhub H_{K} mitnimmt, wodurch die Sperrhülse 8 an einem Startsignalanschlag 105a, der von dem Mechanikhalter 105 gebildet wird, anschlägt und hierdurch ein akustisches und/oder taktiles Signal ausgibt, welches dem Anwender der Vorrichtung signalisiert, dass die Produktausschüttung gestartet wurde. Durch die Verschiebung der Sperrhülse 108 um den Betätigungshub H_{B} wird das Verriegelungsglied 108a für eine Bewegung quer und zu der Längsachse L hin freigegeben, da der Mechanikhalter 105 eine Vertiefung 105d aufweist, welche eine solche Bewegung des Verriegelungsglieds 108a zulässt, wenn die Sperrhülse 8 um den Betätigungshub H_{B} verschoben wurde oder wenn die Nadelschutzhülse 103 in ihrer betätigten Position ist.

Da das Signalglied 1011 noch axialfest mit dem Vortriebsglied 107 verbunden ist, wird es um einen ersten Teilhub H_{S} des Ausschütthubs H_{A} in die Ausschüttrichtung mitgenommen, wobei das Signalglied 1011 in etwa um den ersten Teilhub Hs von dem Signalanschlag 1012b weg bewegt wird. Am Ende des ersten Teilhubs Hs, während dem das erste und zweite Eingriffsglied 1011a, 1011b relativ zu der Sperrhülse 108 bewegt werden, wird das erste Eingriffsglied 1011a aus dem Eingriff mit dem Vortriebsglied 107 gedrückt, wobei gleichzeitig das zweite Eingriffsglied 1011b in die zweite Ausnehmung 108c der Sperrhülse 8 mit einer Bewegung quer zu der Längsachse L und radial von der Längsachse L weg bewegt wird. Hierdurch wird das Signalglied 1011 daran gehindert, sich in die proximale Richtung relativ zu dem Gehäuse 102 oder der Sperrhülse 108 zu bewegen. Das zweite Eingriffsglied 1011b wird von dem Außenumfang des Vortriebsglieds 7 in den Eingriff mit der Ausnehmung 108c gehalten wenn das Vortriebsglied 107 um seinen zweiten Teilhub des Ausschütthubs H_{A} bewegt wird. Die Außenumfangsfläche des Vortriebsglieds 107 hält das zweite Eingriffselement 106b in dem Eingriff mit der ersten Ausnehmung 108b der Sperrhülse 108. Am Ende des Ausschütthubs H_{A} gibt das Vortriebsglied 107 das zweite Eingriffsglied 1011b aus dem Eingriff mit der Sperrhülse 108 frei, wodurch das zweite Eingriffsglied 1011b aus dem Eingriff mit der Ausnehmung 108c bewegt wird, insbesondere zu der Längsachse L hin, so dass die zweite Feder 1010 das Signalglied 1011 entgegen die Ausschüttrichtung, d. h. in die proximale Richtung beschleunigt, so dass beim Auftreffen des Signalglieds 1011 auf dem Signalanschlag 1012b ein akustisches und/oder taktiles Signal erzeugt wird.

Der Eingriff des zweiten Eingriffselements 106b in die erste Ausnehmung 108b bleibt bestehen, wodurch eine Bewegung der Sperrhülse 108 in die distale Richtung relativ zu dem Gehäuse 102 verhindert wird.

Durch Abnehmen des Autoinjektors von der Injektionsstelle kann die zweite Feder 1010 die Schalthülse 1015 und die Nadelschutzhülse 103 aus der betätigten Position in die Nadelschutzposition um den Nadelschutzhub H_{N} bewegen, wobei das Verriegelungsglied 108a aus dem Eingriff mit der Ausnehmung 1015a gedrückt wird, wobei sich die Schalthülse 1015 relativ zu der Sperrhülse 108 in die distale Richtung bewegt. Wenn die Nadelschutzhülse 103 in ihrer Nadelschutzposition ist, verschnappt das Verriegelungsglied 108a mit der Schalthülse 1015, wobei das Verriegelungsglied 108a ein Zurückschieben der Nadelschutzhülse 103 in ihre betätigte Position verhindert. Bei dem Versuch, die Nadelschutzhülse 103 aus der Nadelschutzposition in die betätigte Position zurückzuschieben, stößt das Schaltglied 1015 an dem Verriegelungsglied 108a an, welches die Bewegung der Nadelschutzhülse 103 in die betätigte Position verhindert. Die Sperrhülse 108 stützt sich hierzu axial an dem Startsignalanschlag 105a des Mechanikhalters 105 ab.

### Bezugszeichenliste

- 1: Aufnahmegehäuse
- 2: Produktbehälterhalter
- 3: Nadelschutzhülse
- 4: Feder
- 5: Antriebsgehäuse

- 10: Produktbehälter
- 11: Injektionsnadel
- 12: Produktbehälterabschnitt
- 13: Kolben
- 14: Nadelhalteabschnitt
- 15: Produkt
- 16: Flansch
- 17: Nadelschutzkappe
- 18: Umfangsfläche/Außenfläche
- 19: Spalt

- 20: Kappe
- 21: Hülsenabschnitt
- 22: Eingriffsglied
- 23: Innenhülse
- 24: Abragung

- 25: Entfernerelement
- 26: Eingriffselement
- 27: Koppelabschnitt
- 27a: Ausnehmung
- 28: Verformungsabschnitt
- 29: Arm
- 30: Eingriffsnocke
- 31: erste Wendel
- 32: zweite Wendel
- 33: dritte Wendel
- 34: Verbindungssteg

- 40: Blockierabschnitt
- 41: Freigabeabschnitt/Ausnehmung
- 42: Axialanschlag
- 43: Basisabschnitt

- L: Längsachse

## Patentansprüche

1. Injektionsvorrichtung, umfassend:
- einen Produktbehälter (10) mit einer Injektionsnadel (11),
- eine Nadelschutzkappe (17), die an dem Produktbehälter (10) lösbar befestigt und die Injektionsnadel (11) umschliesst und in Bezug auf die Umgebung steril abdichtet,
- eine Kappe (20), die über ein Entfernerelement (25), welches ein Eingriffselement (26) aufweist, so mit der Nadelschutzkappe (17) verbunden ist, dass das Entfernen der Kappe (20) von der Injektionsvorrichtung das Entfernen der Nadelschutzkappe (17) von dem Produktbehälter (10) bewirkt,
- wobei das Eingriffselement (26) in einer Eingriffsposition in Bezug auf die Nadelschutzkappe (17) ist, wobei in der Eingriffsposition ein Abschnitt der Nadelschutzkappe (17) distal in einer Flucht mit dem Eingriffselement (26) angeordnet ist, wodurch das Eingriffselement (26) gegen ein proximales Ende der Nadelschutzkappe (17) drückt oder dort anschlägt, wenn das Entfernerelement (25) zusammen mit der Kappe (20) relativ zu der Nadelschutzkappe (17) in die distale Richtung bewegt wird, wobei
- die Kappe (20) und das Eingriffselement (26) so gekoppelt sind, dass die Kappe (20) während des Entfernens von der Injektionsvorrichtung relativ zu dem Eingriffselement (26) bewegbar ist oder bewegt wird, **gekennzeichnet dadurch, dass**
- die Injektionsvorrichtung einen Blockierabschnitt (40) aufweist, wobei der Blockierabschnitt (40) mit der Kappe (20) so gekoppelt ist, dass der Blockierabschnitt (40) aus einer Freigabeposition, wobei der Blockierabschnitt in der Freigabeposition proximal des Eingriffselements (26) und/oder des proximalen Endes der Nadelschutzkappe (17) angeordnet ist, in eine Blockierposition bewegbar ist, und sich in der Freigabeposition befindet, wenn die Kappe (20) an einem Aufnahmegehäuse (1) vollständig angebracht oder befestigt ist, und dass das Entfernen der Kappe (20) von dem Aufnahmegehäuse (1) den Blockierabschnitt (40) aus einer Freigabeposition in eine Blockierposition bewegt, wobei der Blockierabschnitt (40) so ausgestaltet ist, dass die Bewegbarkeit des Eingriffselements (26) quer nach aussen nicht blockiert, wenn der Blockierabschnitt (40) in seiner Freigabeposition ist, und wobei der Blockierabschnitt (40) so ausgebildet ist, dass die Bewegbarkeit des Eingriffselements (26) quer nach aussen blockiert, wenn der Blockierabschnitt (40) in seiner Blockierposition ist.

2. Injektionsvorrichtung nach Anspruch 1, **dadurch gekennzeichnet, dass** die Kappe (20) während des Entfernens von der Injektionsvorrichtung zunächst relativ zu dem Eingriffselement (26) bewegbar ist oder bewegt wird und anschliessend das Eingriffselement (26) die Bewegung der Kappe (20) mitmacht, wodurch die Nadelschutzkappe (17) von dem Produktbehälter (10) entfernt oder abgezogen wird.

3. Injektionsvorrichtung nach Anspruch 1 oder 2, **dadurch gekennzeichnet, dass** die Kappe (20) während des Entfernens von der Injektionsvorrichtung mit einer Axialbewegung oder einer kombinierten Axial-Drehbewegung von der Injektionsvorrichtung abnehmbar ist.

4. Injektionsvorrichtung nach einem der Ansprüche 1 bis 3, **dadurch gekennzeichnet, dass** die Kappe (20) an dem Aufnahmegehäuse (1), welches den Produktbehälter umgibt, lösbar befestigt ist.

5. Injektionsvorrichtung nach einem der Ansprüche 1 bis 4, **dadurch gekennzeichnet, dass** der Blockierabschnitt (40) von der Kappe (20) gebildet ist, wobei bevorzugt ist, dass die Kappe (20) eine Ausnehmung (41) aufweist, die in der Freigabeposition des Blockierabschnitts (40) entlang der Längsachse (L) in einer Position auf Höhe des Eingriffselements (26) ist, wobei der Blockierabschnitt (40) durch Entfernen der Kappe (20) in die Blockierposition bewegt wird.

6. Injektionsvorrichtung nach einem der Ansprüche 1 bis 4, **dadurch gekennzeichnet, dass** der Blockierabschnitt (40) von dem Entfernerelement (25) gebildet ist, wobei bevorzugt ist, dass der Blockierabschnitt (40) und das Eingriffselement (26) über einen Verformungsabschnitt (28) verbunden sind, der sich während der Bewegung des Blockierabschnitts (40) aus der Freigabeposition in die Blockierposition verformt.

## Claims

1. An injection device including
a) a product container (10) having an injection needle (11),
b) a needle protection cap (17) which is releasably fixed to the product container (10) and encloses the injection needle (11) and seals it off in relation to the environment in sterile fashion,
c) a cap (20) connected by way of a removal element (25) which has an engagement element (26) to the needle protection cap (17) in such a way that the removal of the cap (20) from the injection device causes removal of the needle protection cap (17) from the product container (10),
d) wherein the engagement element (26) is in an engagement position in relation to the needle protection cap (17), wherein in the engagement position a portion of the needle protection cap (17) is arranged distally in alignment with the engagement element (26), whereby the engagement element (26) presses against or strikes a proximal end of the needle protection cap (17) when the removal element (25) is moved together with the cap (20) relative to the needle protection cap (17) in the distal direction
e) the cap (20) and the engagement element (26) are so coupled that the cap (20) is moveable or moved relative to the engagement element (26) during removal from the injection device,
**characterised in that**
f) the injection device has a blocking portion (40), wherein the blocking portion (40) is so coupled to the cap (20) that the blocking portion (40) is moveable out of a release position wherein the blocking portion (40) is arranged proximal to the engagement element (26) and/or the proximal end of the needle protection cap (17) in the release position, into a blocking position and is in the release position when the cap (20) is completely mounted to or fixed to a receiving housing (1), and that removal of the cap (20) from the receiving housing (1) moves the blocking portion (40) from a release position into a blocking position,
g) wherein the blocking portion (40) is of such a configuration that the mobility of the engagement element (26) transversely outwardly is not blocked when the blocking portion (40) is in its release position and wherein the blocking portion (40) is of such a configuration that the mobility of the engagement element (26) is blocked transversely outwardly when the blocking portion (40) is in its blocking position.

2. An injection device according to claim 1 **characterised in that** the cap (20) is firstly moved or moveable relative to the engagement element (26) during removal from the injection device and then the engagement element (26) moves with the cap (20) whereby the needle protection cap (17) is removed or pulled off the product container (10).

3. An injection device according to claim 1 or claim 2 **characterised in that** the cap (20) can be removed from the injection device with an axial movement or a combined axial and rotary movement during removal from the injection device.

4. An injection device according to one of claims 1 to 3 **characterised in that** the cap (20) is releasably fixed to the receiving housing (1) which surrounds the product container.

5. An injection device according to one of claims 1 to 4 **characterised in that** the blocking portion (40) is formed by the cap (20), wherein it is preferred that the cap (20) has a recess (41) which in the release position of the blocking portion (40) along the longitudinal axis (L) is in a position at the level of the engagement element (26), wherein the blocking portion (40) is moved into the blocking position by removal of the cap (20).

6. An injection device according to one of claims 1 to 4 **characterised in that** the blocking portion (40) is formed by the removal element (25), wherein it is preferred that the blocking portion (40) and the engagement element (26) are connected by way of a deformation portion (28) which deforms during the movement of the blocking portion (40) out of the release position into the blocking position.

## Revendications

1. Dispositif d'injection, comprenant :
a) un contenant de produit (10) avec une aiguille d'injection (11),
b) un capuchon de protection d'aiguille (17), qui est fixé de manière détachable sur le contenant de produit (10) et entoure l'aiguille d'injection (11) et l'étanchéifie de manière stérile vis-à-vis de l'environnement,
c) un capuchon (20), qui est relié au capuchon de protection d'aiguille (17) par l'intermédiaire d'un élément de retrait (25), lequel présente un élément d'engagement (26), de sorte que le dispositif de retrait du capuchon (20) du dispositif d'injection provoque le retrait du capuchon de protection d'aiguille (17) du contenant de produit (10),
d) dans lequel l'élément d'engagement (26) est dans une position d'engagement par rapport au capuchon de protection d'aiguille (17), dans lequel, dans la position d'engagement, une partie du capuchon de protection d'aiguille (17) est alignée distalement avec l'élément d'engagement (26), moyennant quoi l'élément d'engagement (26) appuie ou bute contre une extrémité proximale du capuchon de protection d'aiguille (17) lorsque l'élément d'enlèvement (25) est déplacé avec le capuchon (20) par rapport au capuchon de protection d'aiguille (17) dans la direction distale,
e) dans lequel le capuchon (20) et l'élément d'engagement (26) sont accouplés de sorte que le capuchon (20) peut être déplacé ou est déplacé par rapport à l'élément d'engagement (26) pendant le retrait du dispositif d'injection, **caractérisé en ce que**
f) le dispositif d'injection comprend une section de blocage (40), la section de blocage (40) étant couplée au capuchon (20) de telle sorte que la section de blocage (40) est disposée dans la position de libération depuis une position de libération, la section de blocage étant située dans la position de libération à proximité de l'élément d'engagement (26) et/ou de l'extrémité proximale du capuchon de protection d'aiguille (17), dans une position de blocage, et se trouve dans la position de libération lorsque le capuchon (20) est complètement attaché ou fixé à un boîtier de réception (1), et **en ce que** le retrait du capuchon (20) du boîtier de réception (1) déplace la section de blocage (40) d'une position de libération à une position de blocage,
g) dans lequel la partie de blocage (40) est conçue de sorte que la mobilité de l'élément d'engagement (26) transversalement vers l'extérieur ne se bloque pas lorsque la partie de blocage (40) est dans sa position de libération, et dans lequel la partie de blocage (40) est réalisée de sorte que la mobilité de l'élément d'engagement (26) transversalement vers l'extérieur se bloque lorsque la partie de blocage (40) est dans sa position de blocage.

2. Dispositif d'injection selon la revendication 1, **caractérisé en ce que** le capuchon (20) peut être déplacé ou est déplacé tout d'abord par rapport à l'élément d'engagement (26) pendant le retrait du dispositif d'injection et ensuite l'élément d'engagement (26) entraîne le mouvement du capuchon (20), ce qui a pour effet que le capuchon de protection d'aiguille (17) est retiré ou enlevé du contenant de produit (10).

3. Dispositif d'injection selon la revendication 1 ou 2, **caractérisé en ce que** le capuchon (20) peut être ôté du dispositif d'injection avec un mouvement axial ou un mouvement rotatif axial combiné pendant le retrait du dispositif d'injection.

4. Dispositif d'injection selon l'une quelconque des revendications 1 à 3, **caractérisé en ce que** le capuchon (20) est fixé de manière détachable sur le boîtier de réception (1), lequel entoure le contenant de produit.

5. Dispositif d'injection selon l'une quelconque des revendications 1 à 4, **caractérisé en ce que** la partie de blocage (40) est formée par le capuchon (20), dans lequel il est préférable que le capuchon (20) présente un évidement (41) qui dans la position de libération de la partie de blocage (40) est le long de l'axe longitudinal (L) dans une position à hauteur de l'élément d'engagement (26), dans lequel la partie de blocage (40) est déplacée dans la position de blocage par retrait du capuchon (20).

6. Dispositif d'injection selon l'une quelconque des revendications 1 à 4, **caractérisé en ce que** la partie de blocage (40) est formée par l'élément de retrait (25), dans lequel il est préférable que la partie de blocage (40) et l'élément d'engagement (26) soient reliés par l'intermédiaire d'une partie de déformation (28), qui se déforme pendant le mouvement de la partie de blocage (40) de la position de libération dans la position de blocage.
